# EUROPEAN PATENT APPLICATION

(11) **EP 3 266 875 A1**
(43) Date of publication of application: **10.01.2018**
(21) Application number: 16759041.3
(22) Date of filing: 04.03.2016
(51) Int. Cl.: C12P 21/02, C12N 1/19, C12N 15/09

(54) **METHOD FOR PRODUCING GLUTATHIONE**

(30) Priority: 04.03.2015 JP 2015042909
(71) Applicant: Kaneka Corporation, Osaka-shi, Osaka 530-8288 (JP); National University Corporation Kobe University, Kobe-shi, Hyogo 657-8501 (JP)
(72) Inventor: HARA, Kiyotaka, Kobe-shi Hyogo 657-8501 (JP); KONDO, Akihiko, Kobe-shi Hyogo 657-8501 (JP); IWASAKI, Akira, Takasago-shi Hyogo 676-8688 (JP); IWAMOTO, Yuichi, Takasago-shi Hyogo 676-8688 (JP)
(74) Representative: Hoffmann Eitle
(86) International application number: PCT/JP2016/056845
(87) International publication number: WO 2016/140349

(57) **Abstract**

The present invention aims to provide a yeast that is genetically modified so as to more highly produce glutathione, and a method of producing glutathione utilizing the yeast. The present invention provides a method of producing glutathione, including culturing a yeast whose thiol oxidase activity is increased as compared to the parent strain in a culture medium to produce glutathione, and recovering glutathione from the cultured broth obtained.

SEQUENCE LISTING

<110>  Kaneka Corporation
<110>  National University Corporation, Kobe University

<120>  METHOD FOR PRODUCING GLUTATHIONE

<130>  092444

<150>  JP 2015-042909
<151>  2015-03-04

<160>  18

<170>  PatentIn version 3.5

<210>  1
<211>  189
<212>  PRT
<213>  Artificial Sequence

<220>
<223>  Erv1 protein

<400>  1

Met Lys Ala Ile Asp Lys Met Thr Asp Asn Pro Pro Gln Glu Gly Leu
1               5                   10                  15

Ser Gly Arg Lys Ile Ile Tyr Asp Glu Asp Gly Lys Pro Cys Arg Ser
            20                  25                  30

Cys Asn Thr Leu Leu Asp Phe Gln Tyr Val Thr Gly Lys Ile Ser Asn
        35                  40                  45

Gly Leu Lys Asn Leu Ser Ser Asn Gly Lys Leu Ala Gly Thr Gly Ala
    50                  55                  60

Leu Thr Gly Glu Ala Ser Glu Leu Met Pro Gly Ser Arg Thr Tyr Arg
65                  70                  75                  80

Lys Val Asp Pro Pro Asp Val Glu Gln Leu Gly Arg Ser Ser Trp Thr
                85                  90                  95

Leu Leu His Ser Val Ala Ala Ser Tyr Pro Ala Gln Pro Thr Asp Gln
            100                 105                 110

Gln Lys Gly Glu Met Lys Gln Phe Leu Asn Ile Phe Ser His Ile Tyr
        115                 120                 125

Pro Cys Asn Trp Cys Ala Lys Asp Phe Glu Lys Tyr Ile Arg Glu Asn
    130                 135                 140

Ala Pro Gln Val Glu Ser Arg Glu Glu Leu Gly Arg Trp Met Cys Glu

145                 150                 155                 160

Ala His Asn Lys Val Asn Lys Lys Leu Arg Lys Pro Lys Phe Asp Cys
                165                 170                 175

Asn Phe Trp Glu Lys Arg Trp Lys Asp Gly Trp Asp Glu
            180                 185

<210>  2
<211>  563
<212>  PRT
<213>  Artificial Sequence

<220>
<223>  Ero1 protein

<400>  2

Met Arg Leu Arg Thr Ala Ile Ala Thr Leu Cys Leu Thr Ala Phe Thr
1               5                   10                  15

Ser Ala Thr Ser Asn Asn Ser Tyr Ile Ala Thr Asp Gln Thr Gln Asn
            20                  25                  30

Ala Phe Asn Asp Thr His Phe Cys Lys Val Asp Arg Asn Asp His Val
        35                  40                  45

Ser Pro Ser Cys Asn Val Thr Phe Asn Glu Leu Asn Ala Ile Asn Glu
    50                  55                  60

Asn Ile Arg Asp Asp Leu Ser Ala Leu Leu Lys Ser Asp Phe Phe Lys
65                  70                  75                  80

Tyr Phe Arg Leu Asp Leu Tyr Lys Gln Cys Ser Phe Trp Asp Ala Asn
                85                  90                  95

Asp Gly Leu Cys Leu Asn Arg Ala Cys Ser Val Asp Val Val Glu Asp
            100                 105                 110

Trp Asp Thr Leu Pro Glu Tyr Trp Gln Pro Glu Ile Leu Gly Ser Phe
        115                 120                 125

Asn Asn Asp Thr Met Lys Glu Ala Asp Asp Ser Asp Asp Glu Cys Lys
    130                 135                 140

Phe Leu Asp Gln Leu Cys Gln Thr Ser Lys Lys Pro Val Asp Ile Glu
145                 150                 155                 160

Asp Thr Ile Asn Tyr Cys Asp Val Asn Asp Phe Asn Gly Lys Asn Ala
                165                 170                 175

Val Leu Ile Asp Leu Thr Ala Asn Pro Glu Arg Phe Thr Gly Tyr Gly
            180                 185                 190

Gly Lys Gln Ala Gly Gln Ile Trp Ser Thr Ile Tyr Gln Asp Asn Cys
        195                 200                 205

Phe Thr Ile Gly Glu Thr Gly Glu Ser Leu Ala Lys Asp Ala Phe Tyr
    210                 215                 220

Arg Leu Val Ser Gly Phe His Ala Ser Ile Gly Thr His Leu Ser Lys
225                 230                 235                 240

Glu Tyr Leu Asn Thr Lys Thr Gly Lys Trp Glu Pro Asn Leu Asp Leu
                245                 250                 255

Phe Met Ala Arg Ile Gly Asn Phe Pro Asp Arg Val Thr Asn Met Tyr
            260                 265                 270

Phe Asn Tyr Ala Val Val Ala Lys Ala Leu Trp Lys Ile Gln Pro Tyr
        275                 280                 285

Leu Pro Glu Phe Ser Phe Cys Asp Leu Val Asn Lys Glu Ile Lys Asn
    290                 295                 300

Lys Met Asp Asn Val Ile Ser Gln Leu Asp Thr Lys Ile Phe Asn Glu
305                 310                 315                 320

Asp Leu Val Phe Ala Asn Asp Leu Ser Leu Thr Leu Lys Asp Glu Phe
                325                 330                 335

Arg Ser Arg Phe Lys Asn Val Thr Lys Ile Met Asp Cys Val Gln Cys
            340                 345                 350

Asp Arg Cys Arg Leu Trp Gly Lys Ile Gln Thr Thr Gly Tyr Ala Thr
        355                 360                 365

Ala Leu Lys Ile Leu Phe Glu Ile Asn Asp Ala Asp Glu Phe Thr Lys
    370                 375                 380

Gln His Ile Val Gly Lys Leu Thr Lys Tyr Glu Leu Ile Ala Leu Leu
385                 390                 395                 400

Gln Thr Phe Gly Arg Leu Ser Glu Ser Ile Glu Ser Val Asn Met Phe
                405                 410                 415

Glu Lys Met Tyr Gly Lys Arg Leu Asn Gly Ser Glu Asn Arg Leu Ser

420                 425                 430

Ser Phe Phe Gln Asn Asn Phe Phe Asn Ile Leu Lys Glu Ala Gly Lys
        435                 440                 445

Ser Ile Arg Tyr Thr Ile Glu Asn Ile Asn Ser Thr Lys Glu Gly Lys
    450                 455                 460

Lys Lys Thr Asn Asn Ser Gln Ser His Val Phe Asp Asp Leu Lys Met
465                 470                 475                 480

Pro Lys Ala Glu Ile Val Pro Arg Pro Ser Asn Gly Thr Val Asn Lys
                485                 490                 495

Trp Lys Lys Ala Trp Asn Thr Glu Val Asn Asn Val Leu Glu Ala Phe
            500                 505                 510

Arg Phe Ile Tyr Arg Ser Tyr Leu Asp Leu Pro Arg Asn Ile Trp Glu
        515                 520                 525

Leu Ser Leu Met Lys Val Tyr Lys Phe Trp Asn Lys Phe Ile Gly Val
    530                 535                 540

Ala Asp Tyr Val Ser Glu Glu Thr Arg Glu Pro Ile Ser Tyr Lys Leu
545                 550                 555                 560

Asp Ile Gln

<210>  3
<211>  570
<212>  DNA
<213>  Artificial Sequence

<220>
<223>  ERV1

<400>  3
atgaaagcaa tagataaaat gacggataat ccaccacaag aaggcttaag tgggaggaaa      60

ataatatatg acgaagatgg caaaccttgc cgatcatgta acaccctact tgactttcag     120

tacgtgaccg ggaagatatc taatggcctg aagaacctct catctaacgg taaactagca     180

ggtacggggg ctctcactgg cgaagcttca gagttgatgc ctggctcaag aacatacagg     240

aaggttgacc ctcctgacgt agagcaacta ggtagatctt catggacgct gttacactct     300

gtagctgcca gctatcctgc tcaacctaca gaccaacaga agggtgaaat gaaacagttc     360

ttgaatatct tctcacatat ttatccttgc aactggtgtg ctaaagactt tgaaaaatat     420

atcagagaaa atgcaccaca agttgagtca agagaagaac ttgggaggtg gatgtgtgaa     480

gcccacaata aagtcaataa gaaattgagg aagcccaaat ttgactgtaa tttctgggaa 540

aaaagatgga aggacggctg ggacgaataa 570

<210> 4
<211> 1692
<212> DNA
<213> Artificial Sequence

<220>
<223> ERO1

<400> 4
atgagattaa gaaccgccat tgccacactg tgcctcacgg cttttacatc tgcaacttca 60

aacaatagct acatcgccac cgaccaaaca caaaatgcct ttaatgacac tcacttttgt 120

aaggtcgaca ggaatgatca cgttagtccc agttgtaacg taacattcaa tgaattaaat 180

gccataaatg aaaacattag agatgatctt tcggcgttat taaaatctga tttcttcaaa 240

tactttcggc tggatttata caagcaatgt tcattttggg acgccaacga tggtctgtgc 300

ttaaaccgcg cttgctctgt tgatgtcgta gaggactggg atacactgcc tgagtactgg 360

cagcctgaga tcttgggtag tttcaataat gatacaatga aggaagcgga tgatagcgat 420

gacgaatgta agttcttaga tcaactatgt caaaccagta aaaaacctgt agatatcgaa 480

gacaccatca actactgtga tgtaaatgac tttaacggta aaaacgccgt tctgattgat 540

ttaacagcaa atccggaacg atttacaggt tatggtggta agcaagctgg tcaaatttgg 600

tctactatct accaagacaa ctgttttaca attggcgaaa ctggtgaatc attggccaaa 660

gatgcatttt atagacttgt atccggtttc catgcctcta tcggtactca cttatcaaag 720

gaatatttga acacgaaaac tggtaaatgg gagcccaatc tggatttgtt tatggcaaga 780

atcgggaact ttcctgatag agtgacaaac atgtatttca attatgctgt tgtagctaag 840

gctctctgga aaattcaacc atatttacca gaattttcat tctgtgatct agtcaataaa 900

gaaatcaaaa acaaaatgga taacgttatt tcccagctgg acacaaaaat ttttaacgaa 960

gacttagttt ttgccaacga cctaagtttg actttgaagg acgaattcag atctcgcttc 1020

aagaatgtca cgaagattat ggattgtgtg caatgtgata gatgtagatt gtggggcaaa 1080

attcaaacta ccggttacgc aactgccttg aaaattttgt ttgaaatcaa cgacgctgat 1140

gaattcacca aacaacatat tgttggtaag ttaaccaaat atgagttgat tgcactatta 1200

cagactttcg gtagattatc tgaatctatt gaatctgtta acatgttcga aaaaatgtac 1260

gggaaaaggt taaacggttc tgaaaacagg ttaagctcat tcttccaaaa taacttcttc 1320

aacattttga aggaggcagg caaatcgatt cgttacacca tagagaacat caattccact 1380

aaagaaggaa agaaaaagac taacaattct caatcacatg tatttgatga tttaaaaatg 1440

cccaaagcag aaatagttcc aaggccctct aacggtacag taaataaatg gaagaaagct 1500

tggaatactg aagttaacaa cgttttagaa gcattcagat ttatttatag aagctatttg 1560

gatttaccca ggaacatctg ggaattatct ttgatgaagg tatacaaatt ttggaataaa 1620

ttcatcggtg ttgctgatta cgttagtgag gagacacgag agcctatttc ctataagcta 1680

gatatacaat aa 1692

<210> 5
<211> 483
<212> PRT
<213> Artificial Sequence

<220>
<223> Glr1 protein

<400> 5

Met Leu Ser Ala Thr Lys Gln Thr Phe Arg Ser Leu Gln Ile Arg Thr
1               5                   10                  15

Met Ser Thr Asn Thr Lys His Tyr Asp Tyr Leu Val Ile Gly Gly Gly
            20                  25                  30

Ser Gly Gly Val Ala Ser Ala Arg Arg Ala Ala Ser Tyr Gly Ala Lys
        35                  40                  45

Thr Leu Leu Val Glu Ala Lys Ala Leu Gly Gly Thr Cys Val Asn Val
    50                  55                  60

Gly Cys Val Pro Lys Lys Val Met Trp Tyr Ala Ser Asp Leu Ala Thr
65                  70                  75                  80

Arg Val Ser His Ala Asn Glu Tyr Gly Leu Tyr Gln Asn Leu Pro Leu
                85                  90                  95

Asp Lys Glu His Leu Thr Phe Asn Trp Pro Glu Phe Lys Gln Lys Arg
            100                 105                 110

Asp Ala Tyr Val His Arg Leu Asn Gly Ile Tyr Gln Lys Asn Leu Glu
        115                 120                 125

Lys Glu Lys Val Asp Val Val Phe Gly Trp Ala Arg Phe Asn Lys Asp
    130                 135                 140

Gly Asn Val Glu Val Gln Lys Arg Asp Asn Thr Thr Glu Val Tyr Ser
145                 150                 155                 160

Ala Asn His Ile Leu Val Ala Thr Gly Gly Lys Ala Ile Phe Pro Glu
                165                 170                 175

Asn Ile Pro Gly Phe Glu Leu Gly Thr Asp Ser Asp Gly Phe Phe Arg
            180                 185                 190

Leu Glu Glu Gln Pro Lys Lys Val Val Val Val Gly Ala Gly Tyr Ile
        195                 200                 205

Gly Ile Glu Leu Ala Gly Val Phe His Gly Leu Gly Ser Glu Thr His
    210                 215                 220

Leu Val Ile Arg Gly Glu Thr Val Leu Arg Lys Phe Asp Glu Cys Ile
225                 230                 235                 240

Gln Asn Thr Ile Thr Asp His Tyr Val Lys Glu Gly Ile Asn Val His
                245                 250                 255

Lys Leu Ser Lys Ile Val Lys Val Glu Lys Asn Val Glu Thr Asp Lys
            260                 265                 270

Leu Lys Ile His Met Asn Asp Ser Lys Ser Ile Asp Asp Val Asp Glu
        275                 280                 285

Leu Ile Trp Thr Ile Gly Arg Lys Ser His Leu Gly Met Gly Ser Glu
    290                 295                 300

Asn Val Gly Ile Lys Leu Asn Ser His Asp Gln Ile Ile Ala Asp Glu
305                 310                 315                 320

Tyr Gln Asn Thr Asn Val Pro Asn Ile Tyr Ser Leu Gly Asp Val Val
                325                 330                 335

Gly Lys Val Glu Leu Thr Pro Val Ala Ile Ala Ala Gly Arg Lys Leu
            340                 345                 350

Ser Asn Arg Leu Phe Gly Pro Glu Lys Phe Arg Asn Asp Lys Leu Asp
        355                 360                 365

Tyr Glu Asn Val Pro Ser Val Ile Phe Ser His Pro Glu Ala Gly Ser
    370                 375                 380

Ile Gly Ile Ser Glu Lys Glu Ala Ile Glu Lys Tyr Gly Lys Glu Asn
385                 390                 395                 400

Ile Lys Val Tyr Asn Ser Lys Phe Thr Ala Met Tyr Tyr Ala Met Leu
                405                 410                 415

Ser Glu Lys Ser Pro Thr Arg Tyr Lys Ile Val Cys Ala Gly Pro Asn

420                 425                 430

Glu Lys Val Val Gly Leu His Ile Val Gly Asp Ser Ser Ala Glu Ile
        435                 440                 445

Leu Gln Gly Phe Gly Val Ala Ile Lys Met Gly Ala Thr Lys Ala Asp
    450                 455                 460

Phe Asp Asn Cys Val Ala Ile His Pro Thr Ser Ala Glu Glu Leu Val
465                 470                 475                 480

Thr Met Arg

<210>  6
<211>  1452
<212>  DNA
<213>  Artificial Sequence

<220>
<223>  GLR1

<400>  6
atgctttctg caaccaaaca aacatttaga agtctacaga taagaactat gtccacgaac      60

accaagcatt acgattacct cgtcatcggg ggtggctcag ggggtgttgc ttccgcaaga      120

agagctgcat cttatggtgc gaagacatta ctagttgaag ctaaggctct tggtggtacc      180

tgtgttaacg tgggttgtgt tccgaagaaa gtcatgtggt atgcttctga cctcgctact      240

agagtatccc atgcaaatga atatggatta tatcagaatc ttccattaga taaagagcat      300

ttgactttta attggccaga atttaagcag aaaagggatg cttatgtcca taggttgaac      360

ggtatatacc agaagaattt agaaaaagaa aaagtggatg ttgtatttgg atgggctaga      420

ttcaataagg acggtaatgt tgaagttcag aaaagggata atactactga agtttactcc      480

gctaaccata ttttagttgc gaccggtgga aaggctattt tccccgaaaa cattccaggt      540

ttcgaattag gtactgattc tgatgggttc tttagattgg aagaacaacc taagaaagtt      600

gttgttgttg gcgctggtta tattggtatt gagctagcag gtgtgttcca tgggctggga      660

tccgaaacgc acttggtaat tagaggtgaa actgtcttga gaaaatttga tgaatgcatc      720

cagaacacta ttactgacca ttacgtaaag gaaggcatca acgttcataa actatccaaa      780

attgttaagg tggagaaaaa tgtagaaact gacaaactga aaatacatat gaatgactca      840

aagtccatcg atgacgttga cgaattaatt tggacaattg gacgtaaatc ccatctaggt      900

atgggttcag aaaatgtagg tataaagctg aactctcatg accaaataat tgctgatgaa      960

tatcagaaca ccaatgttcc aaacatttat tctctaggtg acgttgttgg aaaagttgaa     1020

ttgacacctg tcgctattgc agcgggcaga aagctgtcta atagactgtt tggtccagag     1080

aaattccgta atgacaaact agattacgag aacgtcccca gcgtaatttt ctcacatcct 1140

gaagccggtt ccattggtat ttctgagaag gaagccattg aaaagtacgg taaggagaat 1200

ataaaggtct acaattccaa atttaccgcc atgtactatg ctatgttgag tgagaaatca 1260

cccacaagat ataaaattgt ttgtgcggga ccaaatgaaa aggttgtcgg tctgcacatt 1320

gttggtgatt cctctgcaga aatcttgcaa gggttcggtg ttgctataaa gatgggtgcc 1380

actaaggctg atttcgataa ttgtgttgct attcatccga ctagcgcaga agaattggtt 1440

actatgagat ga 1452

<210> 7
<211> 678
<212> PRT
<213> Artificial Sequence

<220>
<223> Gsh1p

<400> 7

Met Gly Leu Leu Ala Leu Gly Thr Pro Leu Gln Trp Phe Glu Ser Arg
1               5                   10                  15

Thr Tyr Asn Glu His Ile Arg Asp Glu Gly Ile Glu Gln Leu Leu Tyr
            20                  25                  30

Ile Phe Gln Ala Ala Gly Lys Arg Asp Asn Asp Pro Leu Phe Trp Gly
        35                  40                  45

Asp Glu Leu Glu Tyr Met Val Val Asp Phe Asp Asp Lys Glu Arg Asn
    50                  55                  60

Ser Met Leu Asp Val Cys His Asp Lys Ile Leu Thr Glu Leu Asn Met
65                  70                  75                  80

Glu Asp Ser Ser Leu Cys Glu Ala Asn Asp Val Ser Phe His Pro Glu
                85                  90                  95

Tyr Gly Arg Tyr Met Leu Glu Ala Thr Pro Ala Ser Pro Tyr Leu Asn
            100                 105                 110

Tyr Val Gly Ser Tyr Val Glu Val Asn Met Gln Lys Arg Arg Ala Ile
        115                 120                 125

Ala Glu Tyr Lys Leu Ser Glu Tyr Ala Arg Gln Asp Ser Lys Asn Asn
    130                 135                 140

Leu His Val Gly Ser Arg Ser Val Pro Leu Thr Leu Thr Val Phe Pro

145                 150                 155                 160

Arg Met Gly Cys Pro Asp Phe Ile Asn Ile Lys Asp Pro Trp Asn His
                165                 170                 175

Lys Asn Ala Ala Ser Arg Ser Leu Phe Leu Pro Asp Glu Val Ile Asn
            180                 185                 190

Arg His Val Arg Phe Pro Asn Leu Thr Ala Ser Ile Arg Thr Arg Arg
        195                 200                 205

Gly Glu Lys Val Cys Met Asn Val Pro Met Tyr Lys Asp Ile Ala Thr
    210                 215                 220

Pro Glu Thr Asp Asp Ser Ile Tyr Asp Arg Asp Trp Phe Leu Pro Glu
225                 230                 235                 240

Asp Lys Glu Ala Lys Leu Ala Ser Lys Pro Gly Phe Ile Tyr Met Asp
                245                 250                 255

Ser Met Gly Phe Gly Met Gly Cys Ser Cys Leu Gln Val Thr Phe Gln
            260                 265                 270

Ala Pro Asn Ile Asn Lys Ala Arg Tyr Leu Tyr Asp Ala Leu Val Asn
        275                 280                 285

Phe Ala Pro Ile Met Leu Ala Phe Ser Ala Ala Ala Pro Ala Phe Lys
    290                 295                 300

Gly Trp Leu Ala Asp Gln Asp Val Arg Trp Asn Val Ile Ser Gly Ala
305                 310                 315                 320

Val Asp Asp Arg Thr Pro Lys Glu Arg Gly Val Ala Pro Leu Leu Pro
                325                 330                 335

Lys Tyr Asn Lys Asn Gly Phe Gly Gly Ile Ala Lys Asp Val Gln Asp
            340                 345                 350

Lys Val Leu Glu Ile Pro Lys Ser Arg Tyr Ser Ser Val Asp Leu Phe
        355                 360                 365

Leu Gly Gly Ser Lys Phe Phe Asn Arg Thr Tyr Asn Asp Thr Asn Val
    370                 375                 380

Pro Ile Asn Glu Lys Val Leu Gly Arg Leu Leu Glu Asn Asp Lys Ala
385                 390                 395                 400

Pro Leu Asp Tyr Asp Leu Ala Lys His Phe Ala His Leu Tyr Ile Arg
                405                 410                 415

Asp Pro Val Ser Thr Phe Glu Glu Leu Leu Asn Gln Asp Asn Lys Thr
            420                 425                 430

Ser Ser Asn His Phe Glu Asn Ile Gln Ser Thr Asn Trp Gln Thr Leu
        435                 440                 445

Arg Phe Lys Pro Pro Thr Gln Gln Ala Thr Pro Asp Lys Lys Asp Ser
    450                 455                 460

Pro Gly Trp Arg Val Glu Phe Arg Pro Phe Glu Val Gln Leu Leu Asp
465                 470                 475                 480

Phe Glu Asn Ala Ala Tyr Ser Val Leu Ile Tyr Leu Ile Val Asp Ser
                485                 490                 495

Ile Leu Thr Phe Ser Asp Asn Ile Asn Ala Tyr Ile His Met Ser Lys
            500                 505                 510

Val Trp Glu Asn Met Lys Ile Ala His His Arg Asp Ala Ile Leu Phe
        515                 520                 525

Glu Lys Phe His Trp Lys Lys Ser Phe Arg Asn Asp Thr Asp Val Glu
    530                 535                 540

Thr Glu Asp Tyr Ser Ile Ser Glu Ile Phe His Asn Pro Glu Asn Gly
545                 550                 555                 560

Ile Phe Pro Gln Phe Val Thr Pro Ile Leu Cys Gln Lys Gly Phe Val
                565                 570                 575

Thr Lys Asp Trp Lys Glu Leu Lys His Ser Ser Lys His Glu Arg Leu
            580                 585                 590

Tyr Tyr Tyr Leu Lys Leu Ile Ser Asp Arg Ala Ser Gly Glu Leu Pro
        595                 600                 605

Thr Thr Ala Lys Phe Phe Arg Asn Phe Val Leu Gln His Pro Asp Tyr
    610                 615                 620

Lys His Asp Ser Lys Ile Ser Lys Ser Ile Asn Tyr Asp Leu Leu Ser
625                 630                 635                 640

Thr Cys Asp Arg Leu Thr His Leu Asp Asp Ser Lys Gly Glu Leu Thr
                645                 650                 655

Ser Phe Leu Gly Ala Glu Ile Ala Glu Tyr Val Lys Lys Asn Lys Pro
            660                 665                 670

Ser Ile Glu Ser Lys Cys
        675

<210>  8
<211>  2037
<212>  DNA
<213>  Artificial Sequence

<220>
<223>  GSH1

<400>  8
   atgggactct tagctttggg cacgcctttg cagtggtttg agtctaggac gtacaatgaa      60
   cacataaggg atgaaggtat cgagcagttg ttgtatattt tccaagctgc tggtaaaaga     120
   gacaatgacc ctctttttgg gggagacgag cttgagtaca tggttgtaga ttttgatgat     180
   aaggagagaa attctatgct cgacgtttgc catgacaaga tactcactga gcttaatatg     240
   gaggattcgt ccctttgtga ggctaacgat gtgagttttc accctgagta tggccggtat     300
   atgttagagg caacaccagc ttctccatat ttgaattacg tgggtagtta cgttgaggtt     360
   aacatgcaaa aaagacgtgc cattgcagaa tataagctat ctgaatatgc gagacaagat     420
   agtaaaaata acttgcatgt gggctccagg tctgtccctt tgacgctgac tgtcttcccg     480
   aggatgggat gccccgactt tattaacatt aaggatccgt ggaatcataa aaatgccgct     540
   tccaggtctc tgtttttacc cgatgaagtc attaacagac atgtcaggtt tcctaacttg     600
   acagcatcca tcaggaccag gcgtggtgaa aaagtttgca tgaatgttcc catgtataaa     660
   gatatagcta ctccagaaac ggatgactcc atctacgatc gagattggtt tttaccagaa     720
   gacaaagagg cgaaactggc ttccaaaccg ggtttcattt atatggattc catgggtttt     780
   ggcatgggct gttcgtgctt acaagtgacc tttcaggcac ccaatatcaa caaggcacgt     840
   tacctgtacg atgcattagt gaattttgca cctataatgc tagccttctc tgccgctgcg     900
   cctgctttta aaggttggct agccgaccaa gatgttcgtt ggaatgtgat atctggtgcg     960
   gtggacgacc gtactccgaa ggaaagaggt gttgcgccat tactacccaa atacaacaag    1020
   aacggatttg gaggcattgc caaagacgta caagataaag tccttgaaat accaaagtca    1080
   agatatagtt cggttgatct tttcttgggt gggtcgaaat tttcaatagg gacttataac     1140
   gacacaaatg tacctattaa tgaaaaagta ttaggacgac tactagagaa tgataaggcg    1200
   ccactggact atgatcttgc taaacatttt gcgcatctct acataagaga tccagtatct    1260
   acattcgaag aactgttgaa tcaggacaac aaaacgtctt caaatcactt tgaaaacatc    1320
   caaagtacaa attggcagac attacgtttt aaacccccca cacaacaagc aaccccggac    1380
   aaaaaggatt ctcctggttg gagagtggaa ttcagaccat ttgaagtgca actattagat 1440
   tttgagaacg ctgcgtattc cgtgctcata tacttgattg tcgatagcat tttgaccttt 1500
   tccgataata ttaacgcata tattcatatg tccaaagtat gggaaaatat gaagatagcc 1560
   catcacagag atgctatcct atttgaaaaa tttcattgga aaaaatcatt tcgcaacgac 1620
   accgatgtgg aaactgaaga ttattctata agcgagattt tccataatcc agagaatggt 1680
   atatttcctc aatttgttac gccaatccta tgccaaaaag ggtttgtaac caaagattgg 1740
   aaagaattaa agcattcttc caaacacgag agactatact attatttaaa gctaatttct 1800
   gatagagcaa gcggtgaatt gccaacaaca gcaaaattct ttagaaattt tgtactacaa 1860
   catccagatt acaaacatga ttcaaaaatt tcaaagtcga tcaattatga tttgctttct 1920
   acgtgtgata gacttaccca tttagacgat tcaaaaggtg aattgacatc ctttttagga 1980
   gctgaaattg cagaatatgt aaaaaaaaat aagccttcaa tagaaagcaa atgttaa 2037

<210> 9
<211> 491
<212> PRT
<213> Artificial Sequence

<220>
<223> Gsh2p

<400> 9

Met Ala His Tyr Pro Pro Ser Lys Asp Gln Leu Asn Glu Leu Ile Gln
1               5                   10                  15

Glu Val Asn Gln Trp Ala Ile Thr Asn Gly Leu Ser Met Tyr Pro Pro
            20                  25                  30

Lys Phe Glu Glu Asn Pro Ser Asn Ala Ser Val Ser Pro Val Thr Ile
        35                  40                  45

Tyr Pro Thr Pro Ile Pro Arg Lys Cys Phe Asp Glu Ala Val Gln Ile
    50                  55                  60

Gln Pro Val Phe Asn Glu Leu Tyr Ala Arg Ile Thr Gln Asp Met Ala
65                  70                  75                  80

Gln Pro Asp Ser Tyr Leu His Lys Thr Thr Glu Ala Leu Ala Leu Ser
                85                  90                  95

Asp Ser Glu Phe Thr Gly Lys Leu Trp Ser Leu Tyr Leu Ala Thr Leu
            100                 105                 110

Lys Ser Ala Gln Tyr Lys Lys Gln Asn Phe Arg Leu Gly Ile Phe Arg

115                 120                 125

Ser Asp Tyr Leu Ile Asp Lys Lys Lys Gly Thr Glu Gln Ile Lys Gln
    130                 135                 140

Val Glu Phe Asn Thr Val Ser Val Ser Phe Ala Gly Leu Ser Glu Lys
145                 150                 155                 160

Val Asp Arg Leu His Ser Tyr Leu Asn Arg Ala Asn Lys Tyr Asp Pro
                165                 170                 175

Lys Gly Pro Ile Tyr Asn Asp Gln Asn Met Val Ile Ser Asp Ser Gly
            180                 185                 190

Tyr Leu Leu Ser Lys Ala Leu Ala Lys Ala Val Glu Ser Tyr Lys Ser
        195                 200                 205

Gln Gln Ser Ser Ser Thr Thr Ser Asp Pro Ile Val Ala Phe Ile Val
    210                 215                 220

Gln Arg Asn Glu Arg Asn Val Phe Asp Gln Lys Val Leu Glu Leu Asn
225                 230                 235                 240

Leu Leu Glu Lys Phe Gly Thr Lys Ser Val Arg Leu Thr Phe Asp Asp
                245                 250                 255

Val Asn Asp Lys Leu Phe Ile Asp Asp Lys Thr Gly Lys Leu Phe Ile
            260                 265                 270

Arg Asp Thr Glu Gln Glu Ile Ala Val Val Tyr Tyr Arg Thr Gly Tyr
        275                 280                 285

Thr Thr Thr Asp Tyr Thr Ser Glu Lys Asp Trp Glu Ala Arg Leu Phe
    290                 295                 300

Leu Glu Lys Ser Phe Ala Ile Lys Ala Pro Asp Leu Leu Thr Gln Leu
305                 310                 315                 320

Ser Gly Ser Lys Lys Ile Gln Gln Leu Leu Thr Asp Glu Gly Val Leu
                325                 330                 335

Gly Lys Tyr Ile Ser Asp Ala Glu Lys Lys Ser Ser Leu Leu Lys Thr
            340                 345                 350

Phe Val Lys Ile Tyr Pro Leu Asp Asp Thr Lys Leu Gly Arg Glu Gly
        355                 360                 365

Lys Arg Leu Ala Leu Ser Glu Pro Ser Lys Tyr Val Leu Lys Pro Gln
    370                 375                 380

Arg Glu Gly Gly Gly Asn Asn Val Tyr Lys Glu Asn Ile Pro Asn Phe
385                 390                 395                 400

Leu Lys Gly Ile Glu Glu Arg His Trp Asp Ala Tyr Ile Leu Met Glu
                405                 410                 415

Leu Ile Glu Pro Glu Leu Asn Glu Asn Asn Ile Ile Leu Arg Asp Asn
            420                 425                 430

Lys Ser Tyr Asn Glu Pro Ile Ile Ser Glu Leu Gly Ile Tyr Gly Cys
        435                 440                 445

Val Leu Phe Asn Asp Glu Gln Val Leu Ser Asn Glu Phe Ser Gly Ser
    450                 455                 460

Leu Leu Arg Ser Lys Phe Asn Thr Ser Asn Glu Gly Gly Val Ala Ala
465                 470                 475                 480

Gly Phe Gly Cys Leu Asp Ser Ile Ile Leu Tyr
                485                 490

<210>  10
<211>  1476
<212>  DNA
<213>  Artificial Sequence

<220>
<223>  GSH2

<400>  10
atggcacact atccaccttc caaggatcaa ttgaatgaat tgatccagga agttaaccaa      60

tgggctatca ctaatggatt atccatgtat cctcctaaat tcgaggagaa cccatcaaat     120

gcatcggtgt caccagtaac tatctatcca accccaattc ctaggaaatg ttttgatgag     180

gccgttcaaa tacaaccggt attcaatgaa ttatacgccc gtattaccca agatatggcc     240

caacctgatt cttatttaca taaaacaact gaagcgttag ctctatcaga ttccgagttt     300

actggaaaac tgtggtctct ataccttgct accttaaaat ctgcacagta caaaaagcag     360

aattttaggc taggtatatt tagatcagat tatttgattg ataagaaaaa gggtactgaa     420

cagattaagc aagtcgagtt taatacagtg tcagtgtcat ttgcaggcct tagcgagaaa     480

gttgatagat tgcactctta tttaaatagg gcaaacaagt acgatcctaa aggaccaatt     540

tataatgatc aaaatatggt catttctgat tcaggatacc ttttgtctaa ggcattggcc     600

aaagctgtgg aatcgtataa gtcacaacaa agttcttcta caactagtga tcctattgtc     660

gcattcattg tgcaaagaaa cgagagaaat gtgtttgatc aaaaggtctt ggaattgaat 720

ctgttggaaa aattcggtac caaatctgtt aggttgacgt ttgatgatgt taacgataaa 780

ttgttcattg atgataaaac gggaaagctt ttcattaggg acacagagca ggaaatagcg 840

gtggtttatt acagaacggg ttacacaacc actgattaca cgtccgaaaa ggactgggag 900

gcaagactat tcctcgaaaa aagtttcgca ataaaggccc cagatttact cactcaatta 960

tctggctcca agaaaattca gcaattgttg acagatgagg gcgtattagg taaatacatc 1020

tccgatgctg agaaaaagag tagtttgtta aaaacttttg tcaaaatata tcccttggat 1080

gatacgaagc ttggcaggga aggcaagagg ctggcattaa gtgagccctc taaatacgtg 1140

ttaaaaccac agcgggaagg tggcggaaac aatgtttata aagaaaatat tcctaatttt 1200

ttgaaaggta tcgaagaacg tcactgggat gcatatattc tcatggagtt gattgaacca 1260

gagttgaatg aaaataatat tatattacgt gataacaaat cttacaacga accaatcatc 1320

agtgaactag gaatttatgg ttgcgttcta tttaacgacg agcaagtttt atcgaacgaa 1380

tttagtggct cattactaag atccaaattt aatacttcaa atgaaggtgg agtggcggca 1440

ggattcggat gtttggacag tattattctt tactag 1476

<210> 11
<211> 1515
<212> PRT
<213> Artificial Sequence

<220>
<223> Ycf1p

<400> 11

Met Ala Gly Asn Leu Val Ser Trp Ala Cys Lys Leu Cys Arg Ser Pro
1               5                   10                  15

Glu Gly Phe Gly Pro Ile Ser Phe Tyr Gly Asp Phe Thr Gln Cys Phe
            20                  25                  30

Ile Asp Gly Val Ile Leu Asn Leu Ser Ala Ile Phe Met Ile Thr Phe
        35                  40                  45

Gly Ile Arg Asp Leu Val Asn Leu Cys Lys Lys Lys His Ser Gly Ile
    50                  55                  60

Lys Tyr Arg Arg Asn Trp Ile Ile Val Ser Arg Met Ala Leu Val Leu
65                  70                  75                  80

Leu Glu Ile Ala Phe Val Ser Leu Ala Ser Leu Asn Ile Ser Lys Glu
                85                  90                  95

Glu Ala Glu Asn Phe Thr Ile Val Ser Gln Tyr Ala Ser Thr Met Leu
            100                 105                 110

Ser Leu Phe Val Ala Leu Ala Leu His Trp Ile Glu Tyr Asp Arg Ser
        115                 120                 125

Val Val Ala Asn Thr Val Leu Leu Phe Tyr Trp Leu Phe Glu Thr Phe
    130                 135                 140

Gly Asn Phe Ala Lys Leu Ile Asn Ile Leu Ile Arg His Thr Tyr Glu
145                 150                 155                 160

Gly Ile Trp Tyr Ser Gly Gln Thr Gly Phe Ile Leu Thr Leu Phe Gln
                165                 170                 175

Val Ile Thr Cys Ala Ser Ile Leu Leu Leu Glu Ala Leu Pro Lys Lys
            180                 185                 190

Pro Leu Met Pro His Gln His Ile His Gln Thr Leu Thr Arg Arg Lys
        195                 200                 205

Pro Asn Pro Tyr Asp Ser Ala Asn Ile Phe Ser Arg Ile Thr Phe Ser
    210                 215                 220

Trp Met Ser Gly Leu Met Lys Thr Gly Tyr Glu Lys Tyr Leu Val Glu
225                 230                 235                 240

Ala Asp Leu Tyr Lys Leu Pro Arg Asn Phe Ser Ser Glu Glu Leu Ser
                245                 250                 255

Gln Lys Leu Glu Lys Asn Trp Glu Asn Glu Leu Lys Gln Lys Ser Asn
            260                 265                 270

Pro Ser Leu Ser Trp Ala Ile Cys Arg Thr Phe Gly Ser Lys Met Leu
        275                 280                 285

Leu Ala Ala Phe Phe Lys Ala Ile His Asp Val Leu Ala Phe Thr Gln
    290                 295                 300

Pro Gln Leu Leu Arg Ile Leu Ile Lys Phe Val Thr Asp Tyr Asn Ser
305                 310                 315                 320

Glu Arg Gln Asp Asp His Ser Ser Leu Gln Gly Phe Glu Asn Asn His
                325                 330                 335

Pro Gln Lys Leu Pro Ile Val Arg Gly Phe Leu Ile Ala Phe Ala Met
            340                 345                 350

Phe Leu Val Gly Phe Thr Gln Thr Ser Val Leu His Gln Tyr Phe Leu
        355                 360                 365

Asn Val Phe Asn Thr Gly Met Tyr Ile Lys Ser Ala Leu Thr Ala Leu
    370                 375                 380

Ile Tyr Gln Lys Ser Leu Val Leu Ser Asn Glu Ala Ser Gly Leu Ser
385                 390                 395                 400

Ser Thr Gly Asp Ile Val Asn Leu Met Ser Val Asp Val Gln Lys Leu
                405                 410                 415

Gln Asp Leu Thr Gln Trp Leu Asn Leu Ile Trp Ser Gly Pro Phe Gln
            420                 425                 430

Ile Ile Ile Cys Leu Tyr Ser Leu Tyr Lys Leu Leu Gly Asn Ser Met
        435                 440                 445

Trp Val Gly Val Ile Ile Leu Val Ile Met Met Pro Leu Asn Ser Phe
    450                 455                 460

Leu Met Arg Ile Gln Lys Lys Leu Gln Lys Ser Gln Met Lys Tyr Lys
465                 470                 475                 480

Asp Glu Arg Thr Arg Val Ile Ser Glu Ile Leu Asn Asn Ile Lys Ser
                485                 490                 495

Leu Lys Leu Tyr Ala Trp Glu Lys Pro Tyr Arg Glu Lys Leu Glu Glu
            500                 505                 510

Val Arg Asn Asn Lys Glu Leu Lys Asn Leu Thr Lys Leu Gly Cys Tyr
        515                 520                 525

Met Ala Val Thr Ser Phe Gln Phe Asn Ile Val Pro Phe Leu Val Ser
    530                 535                 540

Cys Cys Thr Phe Ala Val Phe Val Tyr Thr Glu Asp Arg Ala Leu Thr
545                 550                 555                 560

Thr Asp Leu Val Phe Pro Ala Leu Thr Leu Phe Asn Leu Leu Ser Phe
                565                 570                 575

Pro Leu Met Ile Ile Pro Met Val Leu Asn Ser Phe Ile Glu Ala Ser
            580                 585                 590

Val Ser Ile Gly Arg Leu Phe Thr Phe Phe Thr Asn Glu Glu Leu Gln
        595                 600                 605

Pro Asp Ser Val Gln Arg Leu Pro Lys Val Lys Asn Ile Gly Asp Val
    610                 615                 620

Ala Ile Asn Ile Gly Asp Asp Ala Thr Phe Leu Trp Gln Arg Lys Pro
625                 630                 635                 640

Glu Tyr Lys Val Ala Leu Lys Asn Ile Asn Phe Gln Ala Lys Lys Gly
                645                 650                 655

Asn Leu Thr Cys Ile Val Gly Lys Val Gly Ser Gly Lys Thr Ala Leu
            660                 665                 670

Leu Ser Cys Met Leu Gly Asp Leu Phe Arg Val Lys Gly Phe Ala Thr
        675                 680                 685

Val His Gly Ser Val Ala Tyr Val Ser Gln Val Pro Trp Ile Met Asn
    690                 695                 700

Gly Thr Val Lys Glu Asn Ile Leu Phe Gly His Arg Tyr Asp Ala Glu
705                 710                 715                 720

Phe Tyr Glu Lys Thr Ile Lys Ala Cys Ala Leu Thr Ile Asp Leu Ala
                725                 730                 735

Ile Leu Met Asp Gly Asp Lys Thr Leu Val Gly Glu Lys Gly Ile Ser
            740                 745                 750

Leu Ser Gly Gly Gln Lys Ala Arg Leu Ser Leu Ala Arg Ala Val Tyr
        755                 760                 765

Ala Arg Ala Asp Thr Tyr Leu Leu Asp Asp Pro Leu Ala Ala Val Asp
    770                 775                 780

Glu His Val Ala Arg His Leu Ile Glu His Val Leu Gly Pro Asn Gly
785                 790                 795                 800

Leu Leu His Thr Lys Thr Lys Val Leu Ala Thr Asn Lys Val Ser Ala
                805                 810                 815

Leu Ser Ile Ala Asp Ser Ile Ala Leu Leu Asp Asn Gly Glu Ile Thr
            820                 825                 830

Gln Gln Gly Thr Tyr Asp Glu Ile Thr Lys Asp Ala Asp Ser Pro Leu
        835                 840                 845

Trp Lys Leu Leu Asn Asn Tyr Gly Lys Lys Asn Asn Gly Lys Ser Asn

850                 855                 860

Glu Phe Gly Asp Ser Ser Glu Ser Ser Val Arg Glu Ser Ser Ile Pro
865                 870                 875                 880

Val Glu Gly Glu Leu Glu Gln Leu Gln Lys Leu Asn Asp Leu Asp Phe
                885                 890                 895

Gly Asn Ser Asp Ala Ile Ser Leu Arg Arg Ala Ser Asp Ala Thr Leu
            900                 905                 910

Gly Ser Ile Asp Phe Gly Asp Asp Glu Asn Ile Ala Lys Arg Glu His
        915                 920                 925

Arg Glu Gln Gly Lys Val Lys Trp Asn Ile Tyr Leu Glu Tyr Ala Lys
    930                 935                 940

Ala Cys Asn Pro Lys Ser Val Cys Val Phe Ile Leu Phe Ile Val Ile
945                 950                 955                 960

Ser Met Phe Leu Ser Val Met Gly Asn Val Trp Leu Lys His Trp Ser
                965                 970                 975

Glu Val Asn Ser Arg Tyr Gly Ser Asn Pro Asn Ala Ala Arg Tyr Leu
            980                 985                 990

Ala Ile Tyr Phe Ala Leu Gly Ile  Gly Ser Ala Leu Ala  Thr Leu Ile
        995                 1000                1005

Gln Thr  Ile Val Leu Trp Val  Phe Cys Thr Ile His  Ala Ser Lys
    1010                1015                1020

Tyr Leu  His Asn Leu Met Thr  Asn Ser Val Leu Arg  Ala Pro Met
    1025                1030                1035

Thr Phe  Phe Glu Thr Thr Pro  Ile Gly Arg Ile Leu  Asn Arg Phe
    1040                1045                1050

Ser Asn  Asp Ile Tyr Lys Val  Asp Ala Leu Leu Gly  Arg Thr Phe
    1055                1060                1065

Ser Gln  Phe Phe Val Asn Ala  Val Lys Val Thr Phe  Thr Ile Thr
    1070                1075                1080

Val Ile  Cys Ala Thr Thr Trp  Gln Phe Ile Phe Ile  Ile Ile Pro
    1085                1090                1095

Leu Ser Val Phe Tyr Ile Tyr Tyr Gln Gln Tyr Tyr Leu Arg Thr
    1100                1105                1110

Ser Arg Glu Leu Arg Arg Leu Asp Ser Ile Thr Arg Ser Pro Ile
    1115                1120                1125

Tyr Ser His Phe Gln Glu Thr Leu Gly Gly Leu Ala Thr Val Arg
    1130                1135                1140

Gly Tyr Ser Gln Gln Lys Arg Phe Ser His Ile Asn Gln Cys Arg
    1145                1150                1155

Ile Asp Asn Asn Met Ser Ala Phe Tyr Pro Ser Ile Asn Ala Asn
    1160                1165                1170

Arg Trp Leu Ala Tyr Arg Leu Glu Leu Ile Gly Ser Ile Ile Ile
    1175                1180                1185

Leu Gly Ala Ala Thr Leu Ser Val Phe Arg Leu Lys Gln Gly Thr
    1190                1195                1200

Leu Thr Ala Gly Met Val Gly Leu Ser Leu Ser Tyr Ala Leu Gln
    1205                1210                1215

Ile Thr Gln Thr Leu Asn Trp Ile Val Arg Met Thr Val Glu Val
    1220                1225                1230

Glu Thr Asn Ile Val Ser Val Glu Arg Ile Lys Glu Tyr Ala Asp
    1235                1240                1245

Leu Lys Ser Glu Ala Pro Leu Ile Val Glu Gly His Arg Pro Pro
    1250                1255                1260

Lys Glu Trp Pro Ser Gln Gly Asp Ile Lys Phe Asn Asn Tyr Ser
    1265                1270                1275

Thr Arg Tyr Arg Pro Glu Leu Asp Leu Val Leu Lys His Ile Asn
    1280                1285                1290

Ile His Ile Lys Pro Asn Glu Lys Val Gly Ile Val Gly Arg Thr
    1295                1300                1305

Gly Ala Gly Lys Ser Ser Leu Thr Leu Ala Leu Phe Arg Met Ile
    1310                1315                1320

Glu Ala Ser Glu Gly Asn Ile Val Ile Asp Asn Ile Ala Ile Asn
    1325                1330                1335

Glu Ile Gly Leu Tyr Asp Leu Arg His Lys Leu Ser Ile Ile Pro
    1340                1345                1350

Gln Asp Ser Gln Val Phe Glu Gly Thr Val Arg Glu Asn Ile Asp
    1355                1360                1365

Pro Ile Asn Gln Tyr Thr Asp Glu Ala Ile Trp Arg Ala Leu Glu
    1370                1375                1380

Leu Ser His Leu Lys Glu His Val Leu Ser Met Ser Asn Asp Gly
    1385                1390                1395

Leu Asp Ala Gln Leu Thr Glu Gly Gly Gly Asn Leu Ser Val Gly
    1400                1405                1410

Gln Arg Gln Leu Leu Cys Leu Ala Arg Ala Met Leu Val Pro Ser
    1415                1420                1425

Lys Ile Leu Val Leu Asp Glu Ala Thr Ala Ala Val Asp Val Glu
    1430                1435                1440

Thr Asp Lys Val Val Gln Glu Thr Ile Arg Thr Ala Phe Lys Asp
    1445                1450                1455

Arg Thr Ile Leu Thr Ile Ala His Arg Leu Asn Thr Ile Met Asp
    1460                1465                1470

Ser Asp Arg Ile Ile Val Leu Asp Asn Gly Lys Val Ala Glu Phe
    1475                1480                1485

Asp Ser Pro Gly Gln Leu Leu Ser Asp Asn Lys Ser Leu Phe Tyr
    1490                1495                1500

Ser Leu Cys Met Glu Ala Gly Leu Val Asn Glu Asn
    1505                1510                1515

<210> 12
<211> 4548
<212> DNA
<213> Artificial Sequence

<220>
<223> YCF1

<400> 12
atggctggta atcttgtttc atgggcctgc aagctctgta gatctcctga agggtttgga 60

cctatatcct tttacggtga ctttactcaa tgcttcatcg acggtgtgat cctaaatcta 120

tcagcaattt tcatgataac cttcggtatc agagatttag ttaacctttg caagaaaaaa 180

cactctggca tcaaatatag gcggaattgg attattgtct ctaggatggc actagttctg 240

ttggagatag cgtttgtttc acttgcgtct ttaaatattt ctaaagaaga agcggaaaac 300

tttaccattg taagtcaata tgcttctaca atgttatctt tatttgttgc tttagcctta 360

cactggatag aatacgatag atcagttgta gccaatacgg tacttttatt ctattggctt 420

tttgaaacat tcggtaattt tgctaaacta ataaatattc taattagaca cacctacgaa 480

ggcatttggt attccggaca aacgggtttc atactaacgt tattccaagt aataacatgt 540

gccagtatcc tgttacttga agctcttcca aagaagccgc taatgccaca tcaacacata 600

catcaaactt taacaagaag aaaaccaaat ccatacgata gcgcaaacat attttccagg 660

attaccttct cttggatgtc aggtttgatg aaaactggct atgaaaaata cttagtggaa 720

gcagatttat ataaattacc gaggaacttt agtagtgaag aactctctca aaaattggag 780

aaaaactggg aaaatgagtt gaagcaaaaa tcaaatcctt cattatcatg ggctatatgc 840

agaacttttg gatctaaaat gcttttagcc gcattcttta aagcaattca tgatgttcta 900

gcatttactc aaccacaact actaaggatt ttaatcaagt tcgtcacaga ctataacagt 960

gagagacagg atgaccattc ttctcttcaa gggtttgaaa ataaccaccc acaaaaatta 1020

cccattgtaa gagggttttt gattgcgttt gctatgtttc tggtgggctt tactcagaca 1080

tctgtcctgc atcaatattt cctgaatgtc ttcaacacag gcatgtatat taagagcgcc 1140

ctaacggctt taatatatca aaaatcctta gtgctatcta atgaggcttc tggactttcc 1200

tctaccggtg acattgtcaa tctcatgagt gtggatgttc aaaaattaca agatttaaca 1260

caatggctaa atttaatatg gtcagggcct tttcaaatca ttatttgctt atattctctg 1320

tataagttgt tgggaaattc catgtgggtt ggcgtgatta tactagttat tatgatgcca 1380

ttgaactcat ttttgatgag gatacaaaag aagttgcaaa aatcccagat gaagtacaaa 1440

gatgaaagga cccgtgttat aagtgaaata ctaaacaata ttaaatcttt gaagttatat 1500

gcatgggaga agccttatag ggaaaagcta gaagaagtaa gaaataacaa agagttaaaa 1560

aatcttacaa aactaggatg ttatatggct gtgacaagtt ttcagttcaa tatagtacca 1620

ttccttgttt catgttgtac ctttgctgta tttgtttata ctgaggatag agcattgact 1680

actgacttag ttttccctgc tttgactctg ttcaacctgc tctcattccc actaatgatt 1740

attcctatgg ttttaaattc ttttatcgaa gcttctgttt ctattggtag attatttaca 1800

ttctttacca atgaagagct acaaccagat tcggttcagc gtttaccaaa agtaaaaaat 1860

attggcgatg tagccattaa cattggagat gatgctacct ttttatggca acggaaaccg 1920

gaatacaaag tagccttaaa gaatattaat ttccaagcta aaaaaggaaa tttgacctgt 1980

attgttggta aagttggcag tggtaaaaca gctctattgt catgcatgtt aggtgatcta 2040

ttcagggtta aaggtttcgc caccgttcat ggttctgttg cttatgtttc acaagttcca 2100

tggataatga atggtactgt aaaggaaaac attttatttg ggcatagata cgacgcggaa 2160

ttttacgaaa aaacgatcaa ggcctgtgcg ttaactattg atcttgcaat tttgatggat 2220

ggagataaga cattagttgg cgagaaaggg atctccttat ctggaggaca aaaagctcgt 2280

ttgtctttag caagagcagt ttatgcgaga gctgacactt atttacttga tgatcctttg 2340

gcagctgttg atgaacacgt tgccaggcac ttgatcgaac atgtgttggg tccaaatggt 2400

ttattacata caaaaacgaa ggtattagcc actaataagg tgagcgcgtt atccatcgca 2460

gattctattg cattattaga taatggagaa atcacacagc agggtacata tgatgagatt 2520

acgaaggacg ctgattcgcc attatggaaa ttgctcaaca actatggtaa aaaaaataac 2580

ggtaagtcga atgaattcgg tgactcctct gaaagctcag ttcgagaaag tagtatacct 2640

gtagaaggag agctggaaca actgcagaaa ttaaatgatt tggattttgg caactctgac 2700

gccataagtt taaggagggc cagtgatgca actttgggaa gcatcgattt tggtgacgat 2760

gaaaatattg ctaaaagaga gcatcgtgaa cagggaaaag taaagtggaa catttaccta 2820

gagtacgcta aagcttgcaa cccgaaaagc gtttgtgtat tcatattgtt tattgttata 2880

tcgatgttcc tctctgttat gggtaacgtt tggttgaaac attggtctga agttaatagc 2940

cgctatggat ctaatccaaa tgccgcgcgt tacttggcca tttattttgc acttggtatt 3000

ggttcagcac tggcaacatt aatccagaca atcgttctct gggttttttg taccattcat 3060

gcctccaaat atttacacaa cttgatgaca aactctgtgt tgagagcccc aatgacgttt 3120

tttgaaacaa caccaatcgg tagaattcta aacagattct caaatgacat atacaaagtg 3180

gatgctttat taggaagaac attttctcag tttttcgtca atgcagtgaa agtcacattc 3240

actattacgg ttatctgtgc gacgacatgg caatttatct tcattatcat tccactaagt 3300

gtgttttaca tctactacca gcagtattac ctgagaacat caagggagtt gcgtcgttta 3360

gactctatta ctaggtctcc aatctactct catttccaag agactttggg tggccttgca 3420

acggttagag gttattctca acagaaaagg ttttcccaca ttaatcaatg ccgcattgat 3480

aataacatga gtgcgttcta tccctctatc aatgctaacc gttggctagc atataggttg 3540

gaacttattg gttcaattat cattctaggt gctgcaactt tatccgtttt tagactaaaa 3600

caaggcacat taacggcagg tatggtgggt ttatcattaa gctatgcttt acaaatcact 3660

caaacgttaa attggattgt tagaatgact gtggaagttg aaacgaatat tgtttcagtg 3720

gaaagaataa aggaatatgc tgatttgaag agcgaggcac ctttaatagt tgaaggccac 3780

agaccaccca aagaatggcc gagccagggt gatataaagt ttaataatta ttccactcgt 3840

tataggccgg agcttgatct tgttctgaag cacattaata tacacattaa accaaatgaa 3900

aaagttggta tcgtgggtag aacgggtgcg ggaaaatcct cattaacgct agcattattc 3960

aggatgattg aggctagcga gggaaacatc gtaatcgaca acattgccat caacgagatt 4020

gggttatatg atttgagaca taaattgtca atcatacctc aggattctca agtttttgag 4080

ggcactgttc gtgagaacat tgatcccatt aaccaataca ctgatgaagc tatttggagg 4140

gcattggaac tttctcattt gaaagaacac gtgctatcaa tgagcaatga cggattagat 4200

gcccaactaa ccgaaggtgg tggcaactta agtgttggac aaagacaatt attatgtctt 4260

gcaagagcaa tgttggttcc atcaaagatt ttggtgcttg atgaagccac ggccgcagtc 4320

gacgtggaga cagataaagt cgtccaagag acgattcgta ctgctttcaa ggacagaact 4380

atcttgacca tcgcgcatag actgaacacg ataatggaca gtgatagaat catagtgttg 4440

gacaatggta aagtagccga gtttgactct ccgggccagt tattaagtga taacaaatca 4500

ttgttctatt cactgtgcat ggaggctggt ttggtcaatg aaaattaa 4548

<210> 13
<211> 35
<212> DNA
<213> Artificial Sequence

<220>
<223> ERV1-F

<400> 13
ggccgctagc atgaaagcaa tagataaaat gacgg 35

<210> 14
<211> 33
<212> DNA
<213> Artificial Sequence

<220>
<223> ERV1-R

<400> 14
ggccggatcc ttattcgtcc cagccgtcct tcc 33

<210> 15
<211> 36
<212> DNA
<213> Artificial Sequence

<220>
<223> ERO1-F

<400> 15
ggccgctagc atgagattaa gaaccgccat tgccac 36

<210> 16
<211> 41
<212> DNA
<213> Artificial Sequence

<220>
<223>  ERO1-R

<400>  16
ggccggatcc ttattgtata tctagcttat aggaaatagg c                          41


<210>  17
<211>  37
<212>  DNA
<213>  Artificial Sequence

<220>
<223>  YCF1-F

<400>  17
aaaaggatcc atggctggta atcttgtttc atgggcc                               37


<210>  18
<211>  38
<212>  DNA
<213>  Artificial Sequence

<220>
<223>  YCF1-R

<400>  18
aaaactcgag ttaattttca ttgaccaaac cagcctcc                              38

## Description

### [Technical Field]

The present invention relates to a yeast highly producing glutathione, which is used for pharmaceutical products, foods and the like, and a method of producing glutathione by using the yeast.

### [Background Art]

Glutathione is known to exist as a reduced form or an oxidized form. The reduced glutathione is a peptide consisting of three amino acids, cysteine, glutamic acid and glycine. The oxidized glutathione is a compound in which the thiol groups of two reduced glutathione molecules form a disulfide bond. Glutathione exists in not only human body but also many living bodies such as other animals, plants, microorganisms and the like, and is an important compound for living bodies involved in scavenging action of reactive oxygens, detoxification, amino acid metabolisms and the like. As such, it has attracted attention in pharmaceutical, food and cosmetic industries. In addition, since it has been recently found that oxidized glutathione has effects such as promoting the growth of plants and the like, it has been expected to be used in various fields including agriculture.

Industrially, glutathione is currently produced by a fermentation method using yeast. Since glutathione is accumulated within yeast cells, in actual, it is a widely used method to culture yeasts and extract glutathione from the cultured cells. Accordingly, attempts to increase glutathione content in the cultured cells to improve glutathione producibility have been made, by inducing mutagenesis of yeast used for glutathione production or introducing an enzyme involved in glutathione synthesis using genetic recombination (patent documents 1, 2), or by adding L-glutamic acid, L-cysteine and glycine, which are three kinds of amino acids constituting glutathione, to a culture medium. In recent years, methods accumulating glutathione in oxidized form within yeast cells have also been reported. In non-patent document 1, a strain in which a glutathione reductase gene was destructed and the expression of a glutathione transport enzyme was enhanced resulted in increase of oxidized glutathione and 30% increase of total glutathione (oxidized + reduced glutathiones). Patent document 3 reports a method of increasing total glutathione amount including oxidized glutathione by the expression of glutathione peroxidase.

However, it is required to obtain a yeast more efficiently producing glutathione, in order to industrially produce glutathione at a lower cost.

### [Document List]

### [Patent documents]

Patent document 1: JP S64-51098 A
Patent document 2: JP 2012-213376 A
Patent document 3: JP 2014-64472 A

### [non-patent document]

Non-patent document 1: Nature Chemical Biology 9, 119-125 (2013)

### [SUMMARY OF THE INVENTION]

### [Problems to be Solved by the Invention]

Under the above-mentioned background art, the present invention aims to provide a yeast that is genetically modified so as to more highly produce glutathione, and a method of producing glutathione utilizing the yeast.

### [Means of Solving the Problems]

To solve the aforementioned problem, the present inventors made intensive studies and found that an intracellular amount of glutathione including oxidized glutathione was increased in a yeast strain in which thiol oxidase activity was increased. In addition, when the activity of glutathione reductase, which is involved in reduction of oxidized glutathione, was reduced in the thiol oxidase activity-increased strain, surprisingly, a remarkable increase in glutathione production, namely a significant increase in intracellular glutathione amount (oxidized glutathione + reduced glutathione) was revealed, which resulted in the completion of the present invention.

Furthermore, it was confirmed that glutathione production can be more synergistically improved by increasing the activities of γ-glutamylcysteine synthetase (GSH1) and/or glutathione synthetase (GSH2) and/or glutathione transport enzyme (YCF1) in the thiol oxidase activity-increased strain.

Accordingly, the present invention relates to a method of producing glutathione characterized in culturing a yeast whose thiol oxidase activity is increased.

To be specific, the present invention provides the following.
[1] A method of producing glutathione, comprising culturing a yeast whose thiol oxidase activity is increased as compared to the parent strain in a culture medium to produce glutathione, and recovering glutathione from the cultured broth obtained.
[2] The production method according to the above-mentioned [1], wherein the thiol oxidase is selected from the following (a) - (f) :
   (a) a protein consisting of the amino acid sequence shown by SEQ ID NO: 1 or 2,
   (b) a protein having the amino acid sequence shown by SEQ ID NO: 1 or 2, wherein 1 or plural amino acids are deleted, substituted, inserted and/or added, and having a thiol oxidase activity,
   (c) a protein consisting of an amino acid sequence having 60% or more sequence identity to the amino acid sequence shown by SEQ ID NO: 1 or 2,
   (d) a protein consisting of an amino acid sequence encoded by a nucleotide sequence shown by SEQ ID NO: 3 or 4,
   (e) a protein consisting of an amino acid sequence encoded by a DNA that hybridizes with a DNA having a nucleotide sequence complementary to SEQ ID NO: 3 or 4 under stringent conditions, and,
   (f) a protein consisting of an amino acid sequence encoded by a DNA having the nucleotide sequence shown by SEQ ID NO: 3 or 4 wherein 1 or plural nucleotides are substituted, deleted, inserted and/or added, and having a thiol oxidase activity.
[3] The production method according to the above-mentioned [1] or [2], wherein the yeast is a yeast whose glutathione reductase activity is reduced as compared to the parent strain.
[4] The production method according to the above-mentioned [3], wherein the glutathione reductase is selected from the following (a) - (f):
   (a) a protein consisting of the amino acid sequence shown by SEQ ID NO: 5,
   (b) a protein having the amino acid sequence shown by SEQ ID NO: 5, wherein 1 or plural amino acids are deleted, substituted, inserted and/or added, and having a glutathione reductase activity,
   (c) a protein consisting of an amino acid sequence having 60% or more sequence identity to the amino acid sequence shown by SEQ ID NO: 5,
   (d) a protein consisting of an amino acid sequence encoded by a nucleotide sequence shown by SEQ ID NO: 6,
   (e) a protein consisting of an amino acid sequence encoded by a DNA that hybridizes with a DNA having a nucleotide sequence complementary to SEQ ID NO: 6 under stringent conditions, and,
   (f) a protein consisting of an amino acid sequence encoded by a DNA having the nucleotide sequence shown by SEQ ID NO: 6 wherein 1 or plural nucleotides are substituted, deleted, inserted and/or added, and having a glutathione reductase activity.
[5] The production method according to any of the above-mentioned [1] - [4], wherein the yeast is a yeast in which the expression of γ-glutamylcysteine synthetase and/or glutathione synthetase is enhanced as compared to the parent strain.
[6] The production method according to the above-mentioned [5], wherein the γ-glutamylcysteine synthetase is selected from the following (a) - (f):
   (a) a protein consisting of the amino acid sequence shown by SEQ ID NO: 7,
   (b) a protein having the amino acid sequence shown by SEQ ID NO: 7, wherein 1 or plural amino acids are deleted, substituted, inserted and/or added, and having a γ-glutamylcysteine synthetase activity,
   (c) a protein consisting of an amino acid sequence having 60% or more sequence identity to the amino acid sequence shown by SEQ ID NO: 7,
   (d) a protein consisting of an amino acid sequence encoded by a nucleotide sequence shown by SEQ ID NO: 8,
   (e) a protein consisting of an amino acid sequence encoded by a DNA that hybridizes with a DNA having a nucleotide sequence complementary to SEQ ID NO: 8 under stringent conditions, and,
   (f) a protein consisting of an amino acid sequence encoded by a DNA having the nucleotide sequence shown by SEQ ID NO: 8 wherein 1 or plural nucleotides are substituted, deleted, inserted and/or added, and having a γ-glutamylcysteine synthetase activity.
[7] The production method according to the above-mentioned [5], wherein the glutathione synthetase is selected from the following (a) - (f):
   (a) a protein consisting of the amino acid sequence shown by SEQ ID NO: 9,
   (b) a protein having the amino acid sequence shown by SEQ ID NO: 9, wherein 1 or plural amino acids are deleted, substituted, inserted and/or added, and having a glutathione synthetase activity,
   (c) a protein consisting of an amino acid sequence having 60% or more sequence identity to the amino acid sequence shown by SEQ ID NO: 9,
   (d) a protein consisting of an amino acid sequence encoded by a nucleotide sequence shown by SEQ ID NO: 10,
   (e) a protein consisting of an amino acid sequence encoded by a DNA that hybridizes with a DNA having a nucleotide sequence complementary to SEQ ID NO: 10 under stringent conditions, and, (f) a protein consisting of an amino acid sequence encoded by a DNA having the nucleotide sequence shown by SEQ ID NO: 10 wherein 1 or plural nucleotides are substituted, deleted, inserted and/or added, and having a glutathione synthetase activity.
[8] The production method according to any of the above-mentioned [1] - [7], wherein the yeast is a yeast in which the expression of glutathione transport enzyme is enhanced as compared to the parent strain.
[9] The production method according to the above-mentioned [8], wherein the glutathione transport enzyme is selected from the following (a) - (f):
   (a) a protein consisting of the amino acid sequence shown by SEQ ID NO: 11,
   (b) a protein having the amino acid sequence shown by SEQ ID NO: 11, wherein 1 or plural amino acids are deleted, .substituted, inserted and/or added, and having a glutathione transport enzyme activity,
   (c) a protein consisting of an amino acid sequence having 60% or more sequence identity to the amino acid sequence shown by SEQ ID NO: 11,
   (d) a protein consisting of an amino acid sequence encoded by a nucleotide sequence shown by SEQ ID NO: 12,
   (e) a protein consisting of an amino acid sequence encoded by a DNA that hybridizes with a DNA having a nucleotide sequence complementary to SEQ ID NO: 12 under stringent conditions, and,
   (f) a protein consisting of an amino acid sequence encoded by a DNA having the nucleotide sequence shown by SEQ ID NO: 12 wherein 1 or plural nucleotides are substituted, deleted, inserted and/or added, and having a glutathione transport enzyme activity.
[10] The production method according to any of the above-mentioned [1] - [9], wherein the yeast is a yeast belonging to the genus *Saccharomyces,* the genus *Candida* or the genus *Pichia.*
[11] A method of producing reduced glutathione, comprising reducing oxidized glutathione in the glutathione obtained by the production method according to any of the above-mentioned [1] - [10].
[12] A yeast having artificially modified genes such that the expression of thiol oxidase is enhanced and the expression of γ-glutamylcysteine synthetase and/or glutathione synthetase is enhanced as compared to the parent strain.
[13] A yeast having artificially modified genes such that the expression of thiol oxidase is enhanced and the expression of glutathione transport enzyme is enhanced as compared to the parent strain.
[14] The yeast according to the above-mentioned [12] or [13], wherein the yeast is a yeast belonging to the genus *Saccharomyces,* the genus *Candida* or the genus *Pichia.*

### [Effect of the Invention]

According to the method of the present invention, glutathione can be efficiently produced.

### [Description of Embodiments]

Hereinafter, the method of the present invention is explained in detail.

The present invention is a method of producing glutathione by a yeast in which intracellular thiol oxidase activity is increased, and preferably, glutathione reductase activity is reduced.

More preferably, the present invention is a method of producing glutathione by a yeast whose γ-glutamylcysteine synthetase (GSH1) and/or glutathione synthetase (GSH2) and/or glutathione transport enzyme (YCF1) activities are increased, in addition to the aforementioned properties.

In the present invention, "enzyme activity is increased" means that an enzyme activity of interest is increased as compared to that of the parent strain such as wild-type strain and the like. "Enzyme activity is increased" encompasses not only increasing an enzyme activity of interest in a strain natively having the enzyme activity, but also conferring an enzyme activity of interest to a strain natively lacking the enzyme activity.

Increase of enzyme activity can be accomplished by, for example, artificially modifying a gene of a strain. Such modification can be achieved by, for example, enhancing the expression of a gene encoding an enzyme of interest.

Enhancement of gene expression can be achieved by, for example, substituting the promoter of the gene on chromosome with a more potent promoter. The "more potent promoter" means a promoter that improves transcription of a gene as compared to the naturally occurring wild-type promoter. As a more potent promoter, a highly active form of native promoter may be obtained using various reporter genes. Alternatively, as more potent promoters, known high expression promoters, for example, PGK1, PDC1, TDH3, TEF1, HXT7, ADH1 and the like gene may also be used. The substitution with a more potent promoter can be utilized in combination with the below-mentioned increase of copy number of gene. As an example of utilization of a more potent promoter, a method of enhancing γ-glutamylcysteine synthetase activity by substituting the promoter of the γ-glutamylcysteine synthetase gene on chromosome with a promoter having a potent transcriptional activity is disclosed (Yasuyuki Ohtake et al., Bioscience and Industry, 50(10), 989-994, 1992).

Enhancement of gene expression can be achieved by, for example, increasing copy number of the gene.

The copy number of a gene can be increased by introducing the gene of interest onto the chromosome. Introduction of a gene onto the chromosome can be performed, for example, by utilizing homologous recombination. For example, many copies of a gene can be introduced into the chromosome by homologous recombination using a sequence containing many copies in the chromosome as the target. As the sequence containing many copies in the chromosome, autonomously replicating sequence (ARS) consisting of unique short repeat sequences, and rDNA sequence having about 150 copies can be mentioned. Using a plasmid containing ARS, yeast was transformed as described in WO 95/32289. Alternatively, a gene may be incorporated into a transposon, and the transposon may be transferred into the chromosome to introduce many copies of the gene.

In addition, the copy number of a gene can also be increased by introducing a vector containing the gene of interest into a host. As the vector, for example, a plasmid having a replication origin of CEN 4 or a multiple copy type plasmid having a replication origin of 2 µm DNA can be used preferably. The gene of interest may be inserted into a vector in combination with a suitable promoter to achieve expression of the gene of interest. When a vector containing a promoter suitable for expressing the gene is used, the gene of interest may be expressed by utilizing the promoter in the vector.

In addition, a modification to increase enzyme activity can also be achieved by, for example, enhancing the specific activity of the enzyme of interest. An enzyme having an enhanced specific activity can be obtained by, for example, searching through various organisms. Also, a highly active form may be acquired by introducing mutation into native enzymes. The specific activity may be enhanced solely or enhanced in free combination with the above-mentioned method for enhancing gene expression.

Increase in the enzyme activity of interest can be confirmed by measuring the activity of the enzyme. Thiol oxidase activity can be measured by the method described in FEBS,Letters 477 (2000) 62-66. γ-Glutamylcysteine synthetase activity can be measured by the method of Jackson (Jackson, R. C., Biochem. J., 111, 309 (1969)). Glutathione synthetase activity can be measured by the method of Gushima et al. (Gushima, T. et al., J. Appl. Biochem., 5, 210 (1983)). Glutathione transport enzyme activity can be measured by reference to THE JOURNAL OF BIOLOGICAL CHEMISTRY Vol. 273, No. 50, Issue of December 11, pp. 33449-33454, 1998.

Increase of the transcription amount of a gene encoding the enzyme of interest can be confirmed by comparing the amount of mRNA transcribed from the gene with that of the parent strain. As a method for evaluating the amount of mRNA, Northern hybridization, RT-PCR and the like can be mentioned (Molecular cloning (Cold spring Harbor Laboratory Press, Cold spring Harbor (USA), 2001)). A preferable increase in the amount of mRNA is, for example, not less than 1.5-fold, not less than 2-fold, or not less than 3-fold, as compared to the parent strain.

Increase of the amount of the enzyme of interest can be confirmed by Western blot using an antibody (Molecular cloning (Cold spring Harbor Laboratory Press, Cold spring Harbor (USA), 2001)). The amount of the enzyme of interest preferably increases to, for example, not less than 1.5-fold, not less than 2-fold, or not less than 3-fold, as compared to the parent strain.

When the yeast of the present invention has polyploidy not less than diploid, and the enzyme activity is increased by modification of chromosome, the yeast of the present invention may heterozygously have a chromosome modified to increase enzymatic activity and a wild-type chromosome, or may be a homozygote of a chromosome modified to increase enzymatic activity, as long as it can accumulate glutathione.

In the present invention, "enzyme activity decreases" means that an enzyme activity of interest decreased as compared to that of the parent strain such as wild-type strain and the like, and includes complete disappearance of the activity.

Decrease of enzyme activity can be accomplished by, for example, artificially modifying a gene of a strain. Such modification can be achieved by, for example, mutagenesis treatment or genetic recombination technique.

As a mutagenesis treatment, UV radiation or treatment with mutagens generally used for mutagenesis treatments such as N-methyl-N'-nitro-N-nitrosoguanidine (MNNG), ethylmethane sulfonate (EMS), methylmethane sulfonate (MMS) and the like can be mentioned.

As a genetic recombination technique, known techniques (FEMS Microbiology Letters 165 (1998) 335-340, JOURNAL OF BACTERIOLOGY, Dec. 1995, p7171-7177, Curr Genet 1986; 10(8):573-578, WO 98/14600 etc.) can be utilized.

A modification that decreases the enzyme activity can be achieved by, for example, decreasing the expression of a gene encoding the enzyme of interest. Gene expression can be decreased by, for example, modifying an expression control sequence of the gene such as promoter and the like. When an expression control sequence is modified, preferably not less than one nucleotide, more preferably not less than 2 nucleotides, particularly preferably not less than 3 nucleotides in the expression control sequence are modified. In addition, the expression control sequence may be partly or entirely deleted.

A modification to lower the enzyme activity can be achieved by, for example, partly or entirely deleting the coding region of a gene encoding the enzyme of interest on the chromosome. Furthermore, the whole gene including the sequences before and after the gene on the chromosome may be deleted. The region to be deleted may be any region such as N-terminal region, internal region, C-terminal region and the like as long as the enzyme activity can be decreased. Generally, a longer region to be deleted can certainly inactivate the gene. It is preferable that the sequences before and after the region to be deleted do not have the same reading frame.

A modification to lower the enzyme activity can also be achieved by, for example, introducing amino acid substitution (missense mutation), introducing a termination codon (nonsense mutation), or introducing a frame shift mutation that adds or deletes 1 or 2 nucleotides and the like, into the coding region of a gene encoding the enzyme of interest on the chromosome.

In addition, a modification that lowers the enzyme activity can also be achieved by, for example, introducing other sequence into the coding region of a gene encoding the enzyme of interest on the chromosome. While the insertion site may be any region of the gene, a longer region to be inserted can certainly inactivate the gene. It is preferable that the sequences before and after the region to be inserted do not have the same reading frame. While other sequence is not particularly limited as long as it decreases the function of the protein to be encoded or makes the function disappear, for example, a marker gene and a gene useful for the production of γ-glutamyl compounds such as glutathione and the like can be mentioned.

Modification of a gene on the chromosome as mentioned above can be achieved by, for example, producing a deleted gene lacking a partial sequence of the gene and modified to not produce a protein that functions normally, transforming a yeast with a recombinant DNA containing the deleted gene, and substituting the gene on the chromosome by a deleted gene by causing homologous recombination between the deleted gene and the gene on the chromosome. In this case, the operation is facilitated when the recombinant DNA contains a marker gene according to the phenotype of the host such as auxotrophy and the like. In addition, a strain having a recombinant DNA integrated with the chromosome can be efficiently obtained when the aforementioned recombinant DNA is linearized by cleavage with a restriction enzyme and the like. Even when a protein encoded by the deleted gene is produced, it has a steric structure different from that of a wild-type protein and the function thereof decreases or disappears.

Depending on the structure of the recombinant DNA to be used, homologous recombination sometimes results in the insertion of a wild-type gene and a deleted gene into the chromosome, with the other part of the recombinant DNA (e.g., vector part and marker gene) interposed between them. Since the wild-type gene functions in this state, it is necessary to cause homologous recombination again between the two genes, remove one copy of the gene together with the vector part and the marker gene from the chromosome DNA, and select a sequence maintaining the deleted gene.

Alternatively, for example, a yeast is transformed with a linear DNA containing any sequence provided with, on both ends thereof, upstream and downstream sequences of the substitution target site on the chromosome, to cause homologous recombination at each of the upstream and downstream of the substitution target site, whereby the substitution target site can be substituted by said any sequence in one step. As said any sequence, a sequence containing a marker gene can be used. The marker gene may be removed thereafter where necessary. When the marker gene is to be removed, a sequence for homologous recombination may be added to the both ends of the marker gene to enable efficient removal of the marker gene.

Decrease in the enzyme activity of interest can be confirmed by measuring the activity of the enzyme. The activity of glutathione reductase can be measured by a known method (Glutathione Reductase Assay Kit Product No. 7510-100-K manufactured by Cosmo Bio etc.).

Decrease of the transcription amount of a gene encoding the enzyme of interest can be confirmed by comparing the amount of mRNA transcribed from the gene with that of the parent strain. As a method for evaluating the amount of mRNA, Northern hybridization, RT-PCR and the like can be mentioned (Molecular cloning (Cold spring Harbor Laboratory Press, Cold spring Harbor (USA), 2001)). The amount of mRNA preferably decreases to, for example, not more than 50%, not more than 20%, not more than 10%, not more than 5%, or 0%, as compared to the parent strain.

Decrease of the amount of the enzyme of interest can be confirmed by Western blot using an antibody (Molecular cloning (Cold spring Harbor Laboratory Press, Cold spring Harbor (USA), 2001)). The amount of the enzyme of interest preferably decreases to, for example, not more than 50%, not more than 20%, not more than 10%, not more than 5%, or 0%, as compared to the parent strain.

As a transformation method of yeast, a method generally used for yeast transformation such as protoplast method, KU method (H. Ito et al., J. Bateriol., 153-163 (1983)), KUR method (Fermentation and Industry, vol.43, p.630-637 (1985)), electroporation method (Luis et al., FEMS Micro biology Letters 165 (1998) 335-340), method using carrier DNA (Gietz R.D. and Schiestl R.H., Methods Mol. Cell. Biol. 5:255-269 (1995)) and the like can be adopted. Operations such as yeast spore formation, separation of haploid yeast, and the like are described in "Chemistry and Biology Experimental Line, 31, experiment technique for yeast", the 1st edition, Hirokawa Shoten; "Bio manual series 10, gene experiment method with yeast" the 1st edition, YODOSHA CO., LTD. and the like.

### (1) thiol oxidase

Thiol oxidase is generally an enzyme that catalyzes the reaction of the following formula (1) in vivo.

According to known documents, thiol oxidase is known to be mainly involved in the folding of protein. For example, ERV1, which is one kind of thiol oxidase, activates Mia40 by oxidizing a thiol group contained in Mia40 that introduces a disulfide bond into proteins in the mitochondrial inner membrane (non-patent document, The EMBO Journal (2012) 31, 3169-3182). There has been made no report to date that shows a relationship between thiol oxidase and glutathione production.
[Chem. 1]

Thiol oxidase 4R'C(R)SH + O₂ → 2R'C(R)S-S(R)CR' + 2H₂O (1)

Thiol oxidase in the present invention only needs to have, as mentioned above, an activity to oxidize an intermolecular or intramolecular thiol group of protein or peptide with an oxygen molecule and form a disulfide bond (i.e., thiol oxidase activity), and is not particularly limited. For example, it is preferably any of the following (a) - (f):
(a) a protein consisting of the amino acid sequence shown by SEQ ID NO: 1 or 2,
(b) a protein having the amino acid sequence shown by SEQ ID NO: 1 or 2, wherein 1 or plural amino acids are deleted, substituted, inserted and/or added, and having a thiol oxidase activity,
(c) a protein consisting of an amino acid sequence having 60% or more sequence identity to the amino acid sequence shown by SEQ ID NO: 1 or 2,
(d) a protein consisting of an amino acid sequence encoded by a nucleotide sequence shown by SEQ ID NO: 3 or 4,
(e) a protein consisting of an amino acid sequence encoded by a DNA that hybridizes with a DNA having a nucleotide sequence complementary to SEQ ID NO: 3 or 4 under stringent conditions, and
(f) a protein consisting of an amino acid sequence encoded by a DNA having the nucleotide sequence shown by SEQ ID NO: 3 or 4 wherein 1 or plural nucleotides are substituted, deleted, inserted and/or added, and having a thiol oxidase activity.

A protein having the amino acid sequence shown by SEQ ID NO: 1 or 2, wherein 1 or plural amino acids are substituted, inserted, deleted and/or added can be prepared according to a known method described in "Current Protocols in Molecular Biology (John Wiley and Sons, Inc., 1989)" and the like, and is encompassed in the above-mentioned protein as long as it has a thiol oxidase activity.

An amino acid sequence modified by substitution, insertion, deletion and/or addition may contain only one kind (e.g., substitution) of modification, or two or more kinds of modifications (e.g., substitution and insertion). In the case of substitution, an amino acid used for substitution is preferably an amino acid having properties similar to those of amino acid before substitution (cognate amino acid). As used herein, amino acids in the same group in the following groups are the cognate amino acids.
(group 1: neutral non-polar amino acids) Gly, Ala, Val, Leu, Ile, Met, Cys, Pro, Phe
(group 2: neutral polar amino acids) Ser, Thr, Gln, Asn, Trp, Tyr
(group 3: acidic amino acids) Glu, Asp
(group 4: basic amino acids) His, Lys, Arg

In the above-mentioned description, the plural amino acids mean, for example, not more than 60, preferably 20, more preferably 15, further preferably 10, further preferably 5, 4, 3 or 2 amino acids.

The sequence identity to the amino acid sequence shown in SEQ ID NO: 1 - 2 is preferably not less than 60%, more preferably not less than 70%, further preferably not less than 80%, further preferably not less than 85%, further preferably not less than 90%, most preferably not less than 95%. The sequence identity to the amino acid sequence is expressed by a value obtained by comparing the amino acid sequence shown in SEQ ID NO: 1 - 2 and the amino acid sequence desired to be evaluated, dividing the number of positions, at which amino acids matched between the both sequences, by the total number of the compared amino acids, and multiplying the value by 100.

An additional amino acid sequence can be bonded to the amino acid sequence described in SEQ ID NO: 1 - 2 as long as it has a thiol oxidase activity. In addition, a fusion protein with other protein can also be provided.

The DNA that hybridizes with a DNA having a nucleotide sequence complementary to the nucleotide sequence shown by SEQ ID NO: 3 or 4 under stringent conditions means a DNA obtained by colony hybridization method, plaque hybridization method, or Southern hybridization method and the like under stringent conditions by using a DNA consisting of a nucleotide sequence complementary to the nucleotide sequence shown by SEQ ID NO: 3 or 4 as a probe.

Hybridization can be performed according to the method described in "Molecular Cloning, A laboratory manual, second edition (Cold Spring Harbor Laboratory Press, 1989)" and the like. DNA that hybridizes under stringent conditions is, for example, a DNA obtained by hybridization using a filter immobilizing a colony or plaque-derived DNA in the presence of 0.7 - 1.0 M NaCl at 65°C, and washing the filter with 2-fold concentration of SSC solution (1 x concentration of SSC solution is composed of 150 mM sodium chloride and 15 mM sodium citrate) at 65°C. It is preferably a DNA obtained by washing with 1 x concentration of SSC solution at 65°C, more preferably 0.5 x concentration of SSC solution at 65°C, further preferably 0.2 x concentration of SSC solution at 65°C, most preferably 0.1 x concentration of SSC solution at 65°C.

While the hybridization conditions are described above, they are not particularly limited to those conditions. As factors affecting the stringency of hybridization, plural factors such as temperature, salt concentration and the like are considered, and those of ordinary skill in the art can realize the optimal stringency by appropriately determining those factors.

A DNA hybridizable under the above-mentioned conditions is, for example, a DNA having sequence identity of not less than 7.0%, preferably not less than 74%, more preferably not less than 79%, further preferably not less than 85%, further more preferably not less than 90%, most preferably not less than 95%, to the DNA shown by SEQ ID NO: 3 or 4.

The sequence identity (%) of DNA is expressed by a value obtained by optimally aligning two DNAs to be compared, dividing the number of positions, at which a nucleic acid base (e.g., A, T, C, G, U or I) matched between the both sequences, by the total number of the compared nucleotides, and multiplying the resulting value by 100.

The sequence identity of DNA can be calculated using, for example, the following tools for sequence analysis: GCG Wisconsin Package (Program Manual for The Wisconsin Package, Version 8, September 1994, Genetics Computer Group, 575 Science Drive Medison, Wisconsin, USA 53711; Rice, P. (1996) Program Manual for EGCG Package, Peter Rice, The Sanger Centre, Hinxton Hall, Cambridge, CB10 1RQ, England) and the.e.xPASy World Wide Web Molecule Server for Biology (Geneva University Hospital and University of Geneva, Geneva, Switzerland).

DNA having a nucleotide sequence shown by SEQ ID NO: 3 or 4 wherein 1 or plural nucleotides are substituted, deleted, inserted and/or added can be prepared according to a known method described in "Current Protocols in Molecular Biology (John Wiley and Sons, Inc., 1989)" and the like.

A nucleotide sequence modified by substitution, insertion, deletion and/or addition may contain only one kind (e.g., substitution) of modification, or two or more kinds of modifications (e.g., substitution and insertion).

The plural nucleotides described above are not particularly limited as long as a protein encoded by the DNA has a thiol oxidase activity, and mean, for example, not more than 150, preferably 100, more preferably 50, further preferably 20, further more preferably 10, 5, 4, 3 or 2 nucleotides.

The representative thiol oxidase in the present invention includes thiol oxidase (ERV1) and thiol oxidase (ERO1).

### (2) glutathione reductase

Glutathione reductase is an enzyme having an activity to reduce oxidized glutathione represented by the following formula (2) by utilizing NADPH (reduced nicotinamide dinucleotide phosphate).
[Chem. 2]

Glutathione reductase GS-SG (oxidized glutathione) + NADPH → 2GSH (reduced glutathione) + NADP⁺ (2)

Glutathione reductase in the present invention only needs to have an activity to reduce disulfide bond by utilizing NADPH or NADH (i.e., glutathione reductase activity), and is not particularly limited, and
(a) a protein consisting of the amino acid sequence shown by SEQ ID NO: 5,
(b) a protein having the amino acid sequence shown by SEQ ID NO: 5, wherein 1 or plural amino acids are deleted, substituted, inserted and/or added, and having a glutathione reductase activity,
(c) a protein consisting of an amino acid sequence having 60% or more sequence identity to the amino acid sequence shown by SEQ ID NO: 5,
(d) a protein consisting of an amino acid sequence encoded by a nucleotide sequence shown by SEQ ID NO: 6,
(e) a protein consisting of an amino acid sequence encoded by a DNA that hybridizes with a DNA having a nucleotide sequence complementary to SEQ ID NO: 6 under stringent conditions, and, (f) a protein consisting of an amino acid sequence encoded by a DNA having the nucleotide sequence shown by SEQ ID NO: 6 wherein 1 or plural nucleotides are substituted, deleted, inserted and/or added, and having a glutathione reductase activity
   can be mentioned.

A protein having the amino acid sequence shown by SEQ ID NO: 5, wherein 1 or plural amino acids are substituted, inserted, deleted and/or added can be prepared according to the method described in the above-mentioned (1), and is encompassed in the above-mentioned protein as long as it has a glutathione reductase activity.

An amino acid sequence modified by substitution, insertion, deletion and/or addition may contain only one kind (e.g., substitution) of modification, or two or more kinds of modifications (e.g., substitution and insertion). In the case of substitution, an amino acid used for substitution is preferably an amino acid having properties similar to those of amino acid before substitution (cognate amino acid). Cognate amino acid is as mentioned above in (1).

In the above-mentioned description, the plural amino acids mean, for example, not more than 60, preferably 20, more preferably 15, further preferably 10, further preferably 5, 4, 3 or 2 amino acids.

The sequence identity to the amino acid sequence shown in SEQ ID NO: 5 is preferably not less than 60%, more preferably not less than 70%, further preferably not less than 80%, further preferably not less than 85%, further preferably not less than 90%, most preferably not less than 95%. The sequence identity to the amino acid sequence can be calculated by the aforementioned method in (1).

An additional amino acid sequence can be bonded to the amino acid sequence described in SEQ ID NO: 5 as long as it has a glutathione reductase activity. In addition, a fusion protein with other protein can also be provided.

The DNA that hybridizes with a DNA having a nucleotide sequence complementary to the nucleotide sequence shown by SEQ ID NO: 6 under stringent conditions means a DNA obtained by colony hybridization method, plaque hybridization method, or Southern hybridization method and the like under stringent conditions by using a DNA consisting of a nucleotide sequence complementary to the nucleotide sequence shown by SEQ ID NO: 6 as a probe. The conditions and the like of hybridization are as mentioned above in (1).

A DNA hybridizable under the above-mentioned conditions is, for example, a DNA having sequence identity of not less than 70%, preferably not less than 74%, more preferably not less than 79%, further preferably not less than 85%, further more preferably not less than 90%, most preferably not less than 95%, to the DNA shown by SEQ ID NO: 6. The sequence identity (%) of the DNA is as mentioned in (1) above.

DNA having a nucleotide sequence shown by SEQ ID NO: 6 wherein 1 or plural nucleotides are substituted, deleted, inserted and/or added can be prepared according to the aforementioned method in (1).

A nucleotide sequence modified by substitution, insertion, deletion and/or addition may contain only one kind (e.g., substitution) of modification, or two or more kinds of modifications (e.g., substitution and insertion).

The plural nucleotides described above are not particularly limited as long as a protein encoded by the DNA has a glutathione reductase activity, and mean, for example, not more than 150, preferably 100, more preferably 50, further preferably 20, further more preferably 10, 5, 4, 3 or 2 nucleotides.

A means for confirming that a protein having the amino acid sequence shown by SEQ ID NO: 5, wherein 1 or plural amino acids are deleted, substituted, inserted and/or added, is a protein having a glutathione reductase activity is, for example, a method including producing a transformant that expresses a protein, whose activity is desired to be confirmed, by a DNA recombinant method, producing the protein by using the transformant, placing the protein, oxidized glutathione and NADPH in an aqueous medium, and analyzing the presence or absence of production and accumulation of reduced glutathione or NADP in the aqueous medium by HPLC and the like.

When the yeast of the present invention has polyploidy not less than diploid, the yeast of the present invention may heterozygously have a gene modified to show a decreased enzyme activity and a wild-type gene as long as it can accumulate γ-glutamyl compounds such as glutathione and the like. However, it is generally preferably a homozygote of a gene modified to show a decreased enzymatic activity.

In the present invention, the glutathione reductase activity is preferably decreased to not more than 50%, more preferably not more than 20%, further preferably not more than 10%, particularly preferably not more than 5%, as compared to the parent strain. Substantial disappearance of the glutathione reductase activity is preferable.

### (3) γ-glutamylcysteine synthetase

While γ-glutamylcysteine synthetase in the present invention only needs to have an activity to condense glutamic acid and cysteine to synthesize glutamylcysteine (i.e., γ-glutamylcysteine synthetase activity) and is not particularly limited,
(a) a protein consisting of the amino acid sequence shown by SEQ ID NO: 7,
(b) a protein having the amino acid sequence shown by SEQ ID NO: 7, wherein 1 or plural amino acids are deleted, substituted, inserted and/or added, and having a γ-glutamylcysteine synthetase activity,
(c) a protein consisting of an amino acid sequence having 60% or more sequence identity to the amino acid sequence shown by SEQ ID NO: 7,
(d) a protein consisting of an amino acid sequence encoded by a nucleotide sequence shown by SEQ ID NO: 8,
(e) a protein consisting of an amino acid sequence encoded by a DNA that hybridizes with a DNA having a nucleotide sequence complementary to SEQ ID NO: 8 under stringent conditions, and,
(f) a protein consisting of an amino acid sequence encoded by a DNA having the nucleotide sequence shown by SEQ ID NO: 8 wherein 1 or plural nucleotides are substituted, deleted, inserted and/or added, and having a γ-glutamylcysteine synthetase activity
   can be mentioned.

A protein having the amino acid sequence shown by SEQ ID NO: 7, wherein 1 or plural amino acids are substituted, inserted, deleted and/or added can be prepared according to the method described in the above-mentioned (1), and is encompassed in the above-mentioned protein as long as it has a γ-glutamylcysteine synthetase activity.

An amino acid sequence modified by substitution, insertion, deletion and/or addition may contain only one kind (e.g., substitution) of modification, or two or more kinds of modifications (e.g., substitution and insertion). In the case of substitution, an amino acid used for substitution is preferably an amino acid having properties similar to those of amino acid before substitution (cognate amino acid). Cognate amino acid is as mentioned above in (1).

In the above-mentioned description, the plural amino acids mean, for example, not more than 60, preferably 20, more preferably 15, further preferably 10, further preferably 5, 4, 3 or 2 amino acids.

The sequence identity to the amino acid sequence shown in SEQ ID NO: 7 is preferably not less than 60%, more preferably not less than 70%, further preferably not less than 80%, further preferably not less than 85%, further preferably not less than 90%, most preferably not less than 95%. The sequence identity to the amino acid sequence can be calculated by the aforementioned method in (1).

An additional amino acid sequence can be bonded to the amino acid sequence described in SEQ ID NO: 7 as long as it has a γ-glutamylcysteine synthase activity. In addition, a fusion protein with other protein can also be provided.

The DNA that hybridizes with a DNA having a nucleotide sequence complementary to the nucleotide sequence shown by SEQ ID NO: 8 under stringent conditions means a DNA obtained by colony hybridization method, plaque hybridization method, or Southern hybridization method and the like under stringent conditions by using a DNA consisting of a nucleotide sequence complementary to the nucleotide sequence shown by SEQ ID NO: 8 as a probe. The conditions and the like of hybridization are as mentioned above in (1).

A DNA hybridizable under the above-mentioned conditions is, for example, a DNA having sequence identity of not less than 70%, preferably not less than 74%, more preferably not less than 79%, further preferably not less than 85%, further more preferably not less than 90%, most preferably not less than 95%, to the DNA shown by SEQ ID NO: 8. The sequence identity (%) of the DNA is as mentioned in (1) above.

DNA having a nucleotide sequence shown by SEQ ID NO: 8 wherein 1 or plural nucleotides are substituted, deleted, inserted and/or added can be prepared according to the aforementioned method in (1).

A nucleotide sequence modified by substitution, insertion, deletion and/or addition may contain only one kind (e.g., substitution) of modification, or two or more kinds of modifications (e.g., substitution and insertion).

The plural nucleotides described above are not particularly limited as long as a protein encoded by the DNA has a γ-glutamylcysteine synthetase activity, and mean, for example, not more than 150, preferably 100, more preferably 50, further preferably 20, further more preferably 10, 5, 4, 3 or 2 nucleotides.

A means for confirming that a protein having the amino acid sequence shown by SEQ ID NO: 7, wherein 1 or plural amino acids are deleted, substituted, inserted and/or added, is a protein having a γ-glutamylcysteine synthetase activity is, for example, a method including producing a transformant that expresses a protein, whose activity is desired to be confirmed, by a DNA recombinant method, producing the protein by using the transformant, placing the protein, L-glutamic acid and L-cysteine in an aqueous medium, and analyzing the presence or absence of production and accumulation of γ-glutamylcysteine in the aqueous medium by HPLC and the like.

As the γ-glutamylcysteine synthetase in the present invention, GSH1 shown by SEQ ID NO: 7 is preferable from among the above-mentioned proteins.

### (4) glutathione synthetase

While glutathione synthetase in the present invention only needs to have an activity to condense γ-glutamylcysteine and glycine to synthesize glutathione (i.e., glutathione synthetase activity) and is not particularly limited,
(a) a protein consisting of the amino acid sequence shown by SEQ ID NO: 9,
(b) a protein having the amino acid sequence shown by SEQ ID NO: 9, wherein 1 or plural amino acids are deleted, substituted, inserted and/or added, and having a glutathione synthetase activity,
(c) a protein consisting of an amino acid sequence having 60% or more sequence identity to the amino acid sequence shown by SEQ ID NO: 9,
(d) a protein consisting of an amino acid sequence encoded by a nucleotide sequence shown by SEQ ID NO: 10,
(e) a protein consisting of an amino acid sequence encoded by a DNA that hybridizes with a DNA having a nucleotide sequence complementary to SEQ ID NO: 10 under stringent conditions, and,
(f) a protein consisting of an amino acid sequence encoded by a DNA having the nucleotide sequence shown by SEQ ID NO: 10 wherein 1 or plural nucleotides are substituted, deleted, inserted and/or added, and having a glutathione synthetase activity
   can be mentioned.

A protein having the amino acid sequence shown by SEQ ID NO: 9, wherein 1 or plural amino acids are substituted, inserted, deleted and/or added can be prepared according to the method described in the above-mentioned (1), and is encompassed in the above-mentioned protein as long as it has a glutathione synthetase activity.

An amino acid sequence modified by substitution, insertion, deletion and/or addition may contain only one kind (e.g., substitution) of modification, or two or more kinds of modifications (e.g., substitution and insertion). In the case of substitution, an amino acid used for substitution is preferably an amino acid having properties similar to those of amino acid before substitution (cognate amino acid). Cognate amino acid is as mentioned above in (1).

In the above-mentioned description, the plural amino acids mean, for example, not more than 60, preferably 20, more preferably 15, further preferably 10, further preferably 5, 4, 3 or 2 amino acids.

The sequence identity to the amino acid sequence shown in SEQ ID NO: 9 is preferably not less than 60%, more preferably not less than 70%, further preferably not less than 80%, further preferably not less than 85%, further preferably not less than 90%, most preferably not less than 95%. The sequence identity to the amino acid sequence can be calculated by the aforementioned method in (1).

An additional amino acid sequence can be bonded to the amino acid sequence described in SEQ ID NO: 9 as long as it has a glutathione synthetase activity. In addition, a fusion protein with other protein can also be provided.

The DNA that hybridizes with a DNA having a nucleotide sequence complementary to the nucleotide sequence shown by SEQ ID NO: 10 under stringent conditions means a DNA obtained by colony hybridization method, plaque hybridization method, or Southern hybridization method and the like under stringent conditions by using a DNA consisting of a nucleotide sequence complementary to the nucleotide sequence shown by SEQ ID NO: 10 as a probe. The conditions and the like of hybridization are as mentioned above in (1).

A DNA hybridizable under the above-mentioned conditions is, for example, a DNA having sequence identity of not less than 70%, preferably not less than 74%, more preferably not less than 79%, further preferably not less than 85%, further more preferably not less than 90%, most preferably not less than 95%, to the DNA shown by SEQ ID NO: 10. The sequence identity (%) of the DNA is as mentioned in (1) above.

DNA having a nucleotide sequence shown by SEQ ID NO: 10 wherein 1 or plural nucleotides are substituted, deleted, inserted and/or added can be prepared according to the aforementioned method in (1).

A nucleotide sequence modified by substitution, insertion, deletion and/or addition may contain only one kind (e.g., substitution) of modification, or two or more kinds of modifications (e.g., substitution and insertion).

The plural nucleotides described above are not particularly limited as long as a protein encoded by the DNA has a glutathione synthetase activity, and mean, for example, not more than 150, preferably 100, more preferably 50, further preferably 20, further more preferably 10, 5, 4, 3 or 2 nucleotides.

A means for confirming that a protein having the amino acid sequence shown by SEQ ID NO: 9, wherein 1 or plural amino acids are deleted, substituted, inserted and/or added, is a protein having a glutathione synthetase activity is, for example, a method including producing a transformant that expresses a protein, whose activity is desired to be confirmed, by a DNA recombinant method, producing the protein by using the transformant, placing the protein, γ-glutamylcysteine and glycine in an aqueous medium, and analyzing the presence or absence of production and accumulation of glutathione in the aqueous medium by HPLC and the like.

As the glutathione synthetase in the present invention, GSH2 shown by SEQ ID NO: 9 is preferable.

### (5) glutathione transport enzyme

In the present invention, the glutathione transport enzyme means a protein having a function to transport glutathione in the cytoplasm to vacuole (i.e., glutathione transport enzyme activity), and is not particularly limited as long as it has such function.

More preferably, as the glutathione transport enzyme,
(a) a protein consisting of the amino acid sequence shown by SEQ ID NO: 11,
(b) a protein having the amino acid sequence shown by SEQ ID NO: 11, wherein 1 or plural amino acids are deleted, substituted, inserted and/or added, and having a glutathione transport enzyme activity,
(c) a protein consisting of an amino acid sequence having 60% or more sequence identity to the amino acid sequence shown by SEQ ID NO: 11,
(d) a protein consisting of an amino acid sequence encoded by a nucleotide sequence shown by SEQ ID NO: 12,
(e) a protein consisting of an amino acid sequence encoded by a DNA that hybridizes with a DNA having a nucleotide sequence complementary to SEQ ID NO: 12 under stringent conditions, and,
(f) a protein consisting of an amino acid sequence encoded by a DNA having the nucleotide sequence shown by SEQ ID NO: 12 wherein 1 or plural nucleotides are substituted, deleted, inserted and/or added, and having a glutathione transport enzyme activity
   can be mentioned.

A protein having the amino acid sequence shown by SEQ ID NO: 11, wherein 1 or plural amino acids are substituted, inserted, deleted and/or added can be prepared according to the method described in the above-mentioned (1), and is encompassed in the above-mentioned protein as long as it has a glutathione synthetase activity.

An amino acid sequence modified by substitution, insertion, deletion and/or addition may contain only one kind (e.g., substitution) of modification, or two or more kinds of modifications (e.g., substitution and insertion). In the case of substitution, an amino acid used for substitution is preferably an amino acid having properties similar to those of amino acid before substitution (cognate amino acid). Cognate amino acid is as mentioned above in (1).

In the above-mentioned description, the plural amino acids mean, for example, not more than 60, preferably 20, more preferably 15, further preferably 10, further preferably 5, 4, 3 or 2 amino acids.

The sequence identity to the amino acid sequence shown in SEQ ID NO: 11 is preferably not less than 60%, more preferably not less than 70%, further preferably not less than 80%, further preferably not less than 85%, further preferably not less than 90%, most preferably not less than 95%. The sequence identity to the amino acid sequence can be calculated by the aforementioned method in (1).

An additional amino acid sequence can be bonded to the amino acid sequence described in SEQ ID NO: 11 as long as it has a glutathione transport enzyme activity. In addition, a fusion protein with other protein can also be provided.

The DNA that hybridizes with a DNA having a nucleotide sequence complementary to the nucleotide sequence shown by SEQ ID NO: 12 under stringent conditions means a DNA obtained by colony hybridization method, plaque hybridization method, or Southern hybridization method and the like under stringent conditions by using a DNA consisting of a nucleotide sequence complementary to the nucleotide sequence shown by SEQ ID NO: 12 as a probe. The conditions and the like of hybridization are as mentioned above in (1).

A DNA hybridizable under the above-mentioned conditions is, for example, a DNA having sequence identity of not less than 70%, preferably not less than 74%, more preferably not less than 79%, further preferably not less than 85%, further more preferably not less than 90%, most preferably not less than 95%, to the DNA shown by SEQ ID NO: 12. The sequence identity (%) of the DNA is as mentioned in (1) above.

DNA having a nucleotide sequence shown by SEQ ID NO: 12 wherein 1 or plural nucleotides are substituted, deleted, inserted and/or added can be prepared according to the aforementioned method in (1).

A nucleotide sequence modified by substitution, insertion, deletion and/or addition may contain only one kind (e.g., substitution) of modification, or two or more kinds of modifications (e.g., substitution and insertion).

The plural nucleotides described above are not particularly limited as long as a protein encoded by the DNA has a glutathione transport enzyme activity, and mean, for example, not more than 150, preferably 100, more preferably 50, further preferably 20, further more preferably 10, 5, 4, 3 or 2 nucleotides.

As the glutathione transport enzyme in the present invention, YCF1 shown by SEQ ID NO: 11 is preferable.

### (6) yeast of the present invention

The yeast of the present invention is not particularly limited as long as it has glutathione producibility. For example, yeasts belonging to the genus *Saccharomyces* such as *Saccharomyces cerevisiae, Saccharomyces carlesbergensis, Saccharomyces fragilis, Saccharomyces rouxii* and the like, the genus *Candida* such as *Candida utilis, Candida tropicalis* and the like, the genus *Schizosaccaromyces* such as *Schizosaccaromyces pombe* and the like, the genus *Toluropsis* such as *Toluropsis versatilis, Toluropsis petrophilum* and the like, the genus *Pichia,* the genus *Brettanomyces,* the genus *Mycotorula,* the genus *Rhodotorula,* the genus *Hansenula,* the genus *Endomyces* and the like can be mentioned.

Of these, a yeast belonging to the genus *Saccharomyces,* the genus *Candida,* or the genus *Pichia* is preferable, and further, *Saccharomyces cerevisiae* belonging to the genus *Saccharomyces* or *Candida utilis* belonging to the genus *Candida* is preferable.

### (7) Production method of glutathione in the present invention

The yeast of the present invention can be cultured in the same manner as in the general culture of microorganisms. That is, any of synthesis medium, semisynthesis medium and natural (composite) medium can be used as long as it appropriately contains carbon source, nitrogen source, inorganic substance, other nutritions and the like.

As the carbon source, various carbohydrate materials such as glucose, glycerol, fructose, sucrose, maltose, mannose, mannitol, xylose, galactose, starch, starch hydrolysate solution, molasses and the like can be used. In addition, various organic acids such as pyruvic acid, acetic acid, lactic acid and the like, and various amino acids such as aspartic acid, alanine and the like can also be used.

As the nitrogen source, various inorganic and organic ammonium salts such as ammonia or ammonium chloride, ammonium phosphate, ammonium sulfate, ammonium nitrate, ammonium carbonate, ammonium acetate and the like, urea and other nitrogen-containing compounds, as well as nitrogenous organic substances such as peptone, NZ amine, meat extract, yeast extract, corn steep liquor, casein hydrolysate, fish meal or digest thereof, defatted soybean or digest and hydrolysate thereof, and the like, and various amino acids such as aspartic acid, glutamic acid, threonine and the like can be used.

As the inorganic substance, moreover, potassium monohydrogen phosphate, potassium dihydrogen phosphate, magnesium sulfate, sodium chloride, ferrous sulfate, manganese sulfate, calcium carbonate and the like can be used.

Culturing is performed under aerobic culture conditions such as shaking culture, aeration-agitation submerged culture and the like. During culturing, pH is preferably adjusted to 3.0 - 8.0, more preferably 4.0 - 6.0, most preferably 5.0. As a neutralizing agent for pH control, aqueous ammonia, sodium hydroxide, ammonium carbonate and the like can be used. The amount of air supply to the medium in aerobic culture is preferably not less than 0.2 L/min, more preferably not less than 0.5 L/min, further preferably not less than 1 L/min, per 1 L of the medium. When a jar fermentor with a total volume of 2 L is used for culturing, aerobic culture conditions include the above-mentioned quantity of airflow, and the number of stirring of preferably not less than 200 rpm, more preferably not less than 300 rpm, further preferably not less than 400 rpm. The temperature during culturing is 20 - 45°C, preferably 25 - 35°C, most preferably 28 - 32°C. The culture period is generally 16 hr - 72 hr, preferably 24 hr - 48 hr. The initial concentration of the cell in the medium (inoculated concentration) varies depending on the kind of the yeast, medium composition and the like, and the initial turbidity (OD600) is preferably 0.01 - 2.0, more preferably 0.02 - 1.0, further preferably 0.1 - 0.4. As for the above-mentioned carbon source such as glucose and the like, both a batch type including charging them all at once in an initial stage of culture and performing culturing, and a fed-batch type including adding them by small portions throughout the culture period are applicable. According to the study results by the present inventors, the fed-batch type is more preferable since it improves the growth rate, makes the final cell concentration higher, and increases the activities of intracellular glutathione synthesis-related enzymes, though subject to change depending on the microorganism to be used. As an index for determining the feeding rate of the carbon source such as glucose and the like in the fed-batch type, turbidity of the cultured broth, oxygen consumption rate, carbon dioxide generation rate, consumption amount of neutralizing agent for pH adjustment and the like can be utilized effectively. By selecting an appropriate index for the yeast to be used, and changing the feeding rate of the carbon source in response to the change, the yeast culture can be optimized and the final cell concentration and the activity of glutathione synthesis-related enzymes can be improved, as a result of which the producibility of glutathione can be actually improved.

The present method affords a cultured broth composed of a yeast mainly containing a high concentration of glutathione in the cells and medium components. The yeast cells in the cultured broth are separated from the medium components by filtration and centrifugation operations, and glutathione can be extracted from the obtained yeast cells by hot water extraction, alkali extraction method, enzymatic degradation method, autodigestion method, mechanical disruption operation and the like as necessary in an appropriately combination. In addition, the above-mentioned extraction operation may be directly applied to the cultured broth after culturing, or enzymatic degradation method, autodigestion method, or mechanical disruption operation may be performed to elute glutathione in the cells into the medium, and glutathione may be extracted by the above-mentioned extraction operation. A fraction highly containing glutathione or a glutathione powder can be obtained from the thus-obtained glutathione extract by purifying glutathione according to a general method.

It is also possible to obtain glutathione containing a reduced type at a higher proportion by once reducing oxidized glutathione contained in the above-mentioned glutathione extract or the glutathione powder. While the reduction method is not particularly limited, reduction by glutathione reductase is preferably mentioned.

The present invention is explained in more detail in the following by referring to Examples and the like, which are not to be construed as limitative.

### [Examples]

### [Production Example 1] Yeast whose expression of GSH1 gene and GSH2 gene is enhanced (GSH1-enhanced + GSH2-enhanced strain)

*Saccharomyces cerevisiae* YPH499/GSH1, GSH2 strain in which GSH1 and GSH2 are enhanced, which is described in a non-patent document (Hara KY, Kiriyama K, Inagaki A, Nakayama H, Kondo A. (2012) Improvement of glutathione production by metabolic engineering the sulfate assimilation pathway of Saccharomyces cerevisiae. Appl Microbiol Biotechnol, 426(2):129-33.), was used.

### [Production Example 2] Preparation of yeast in which expression of GSH1 gene and GSH2 gene is enhanced and expression of ERV1 gene or ERO1 gene is enhanced (GSH1-enhanced + GSH2-enhanced + ERV1-enhanced strain, GSH1-enhanced + GSH2-enhanced + ERO1-enhanced strain)

Using genomic DNA of *Saccharomyces cerevisiae* YPH499 strain as a template and 5'-GGCCGCTAGCATGAAAGCAATAGATAAAATGACGG-3' (SEQ ID NO: 13) and 5'-GGCCGGATCCTTATTCGTCCCAGCCGTCCTTCC-3' (SEQ ID NO: 14) as primers, PCR amplification was performed, and the amplification product was digested with NheI and BamHI to give a thiol oxidase (ERV1) gene NheI-BamHI fragment. The fragment was ligated to the NheI-BamHI digestion site of pGK406 described in a non-patent document (J. Biochem. (2009)145: 701-708) to give ERV1-expression plasmid pGK406-ERV1. The obtained plasmid pGK406-ERV1 was digested with restriction enzyme NcoI. *Saccharomyces cerevisiae* YPH499/GSH1, GSH2 strain shown in Production Example 1 as host yeast strain was transformed to give transformant YPH499/GSH1, GSH2, ERV1 strain (GSH1-enhanced + GSH2-enhanced + ERV1-enhanced strain).

Using genomic DNA of *Saccharomyces cerevisiae* YPH499 strain as a template and 5'-GGCCGCTAGCATGAGATTAAGAACCGCCATTGCCAC-3' (SEQ ID NO: 15) and 5'-GGCCGGATCCTTATTGTATATCTAGCTTATAGGAAATAGGC-3' (SEQ ID NO: 16) as primers, PCR amplification was performed, and the amplification product was digested with NheI and BamHI to give a thiol oxidase (ERO1) gene NheI-BamHI fragment. The fragment was ligated to the NheI-BamHI digestion site of pGK406 described in a non-patent document (J. Biochem. (2009)145: 701-708) to give ERO1-expression plasmid pGK406-ERO1. The obtained plasmid pGK406-ERO1 was digested with restriction enzyme NcoI. *Saccharomyces cerevisiae* YPH499/GSH1, GSH2 strain shown in Production Example 1 as host yeast strain was transformed to give transformant YPH499/GSH1, GSH2, ERO1 strain (GSH1-enhanced + GSH2-enhanced + ERO1-enhanced strain).

### [Production Example 3] Preparation of strain in which expression of GSH1 gene and GSH2 gene is enhanced, and GLR1 gene is destructed (GSH1-enhanced + GSH2-enhanced + GLR1-destructed strain)

*Saccharomyces cerevisiae* YPH499/ΔGLR1, GSH1, GSH2 strain, in which expression of GSH1 gene and GSH2 gene is enhanced, and GLR1 gene was destructed (GSH1-enhanced + GSH2-enhanced + GLR1-destructed strain), was obtained by the method described in a non-patent document (Kiriyama K, Hara KY, Kondo A. (2013) Oxidized glutathione fermentation using Saccharomyces cerevisiae engineered for glutathione metabolism. ApplMicrobiol Biotechnol, 97(16):7399-7404.).

### [Production Example 4] Preparation of strain in which expression of GSH1 gene and GSH2 gene is enhanced, expression of GLR1 gene is decreased, and expression of ERV1 gene or ERO1 gene is enhanced (GSH1-enhanced + GSH2-enhanced + GLR1-destructed + ERV1-enhanced strain, GSH1-enhanced + GSH2-enhanced + GLR1-destructed + ERO1-enhanced strain)

ERV1-expression plasmid pGK406-ERV1 and ERO1-expression plasmid pGK406-ERO1 shown in Production Example 2 were digested with restriction enzyme NcoI. *Saccharomyces cerevisiae* YPH499/ΔGLR1, GSH1, GSH2 strain shown in Production Example 3 as host yeast strain was transformed to give transformant YPH499/ΔGLR1, GSH1, GSH2, ERV1 strain (GSH1-enhanced + GSH2-enhanced + GLR1-destructed + ERV1-enhanced strain) and YPH499/ΔGLR1, GSH1, GSH2, ERO1 strain (GSH1-enhanced + GSH2-enhanced + GLR1-destructed + ERO1-enhanced strain), respectively.

### [Production Example 5] Preparation of strain in which expression of ERV1 is enhanced, expression of GSH1, GSH2 and YCF1 is further enhanced and GLR1 gene is destructed (GSH1-enhanced + GSH2-enhanced + GLR1-destructed + ERV1-enhanced + YCF1-enhanced strain), and strain in which expression of GSH1, GSH2 and YCF1 is enhanced, and GLR1 gene is destructed (GSH1-enhanced + GSH2-enhanced + GLR1-destructed + YCF1-enhanced strain)

Using genomic DNA of *Saccharomyces cerevisiae* YPH499 strain as a template and 5'-AAAAGGATCCATGGCTGGTAATCTTGTTTCATGGGCC-3' (SEQ ID NO: 17) and 5'-AAAACTCGAGTTAATTTTCATTGACCAAACCAGCCTCC-3' (SEQ ID NO: 18) as primers, PCR amplification was performed, and the amplification product was digested with BamHI and XhoI to give a glutathione transport enzyme (YCF1) gene BamHI-XhoI fragment. The fragment was ligated to the BamHI-XhoI digestion site of p427TEF (manufactured by Cosmo Bio) to give YCF1 expression plasmid p427-YCF1. *Saccharomyces cerevisiae* YPH499/ΔGLR1, GSH1, GSH2, ERV1 strain shown in Production Example 4, and *Saccharomyces cerevisiae* YPH499/ΔGLR1, GSH1, GSH2 strain shown in Production Example 3 as host yeast strains were transformed with p427TEF-YCF1 to give transformant YPH499/ΔGLR1, GSH1, GSH2, ERV1, YCF1 strain (GSH1-enhanced + GSH2-enhanced + GLR1-destructed + ERV1-enhanced + YCF1-enhanced strain) and YPH499/ΔGLR1, GSH1, GSH2, YCF1 strain (GSH1-enhanced + GSH2-enhanced + GLR1-destructed + YCF1-enhanced strain), respectively.

### [Example 1] Production of glutathione by yeast in which expression of GSH1 gene and GSH2 gene is enhanced, and expression of ERV1 gene or ERO1 gene is further enhanced (GSH1-enhanced + GSH2-enhanced + ERV1-enhanced strain, GSH1-enhanced + GSH2-enhanced +ERO1-enhanced strain)

Recombinant yeasts YPH499/GSH1, GSH2, ERV1 and YPH499/GSH1, GSH2, ERO1, in which expression of GSH1 and GSH2 is enhanced and expression of ERV1 or ERO1 is enhanced, which were obtained in Production Example 2, were subjected to seed culture by shaking in SD medium (6.7 g/L yeast nitrogen base w/o amino acids (manufactured by Difco laboratories), 20 g/L glucose) (5 ml) at 30°C for 16 - 24 hr.

Then, they were inoculated to erlenmeyer flask with baffles containing YPD medium (10 g/L yeast extract (manufactured by Difco laboratories), 20 g/L polypeptone (manufactured by Wako Pure Chemical Industries, Ltd.), 20 g/L glucose (manufactured by Nacalai Tesque)) (20 ml) at OD600=0.03, cultured under conditions of 30°C, agitation 150 rpm for 24 hr, and 1 ml of the cultured broth was recovered.

The cells collected by centrifugation were rinsed twice with sterilized water, and heat treated at 95°C for 3 min to elute intracellular glutathione. The glutathione concentration of the supernatant centrifuged at 25°C, 20000xg for 5 min was analyzed by the HPLC method and the glutathione concentration per medium was determined.

The cell concentration was determined by measuring the absorbance at 600 nm, or calculated by standing the cultured broth at 80°C for not less than 12 hr, measuring the weight, determining the dry cell weight by subtracting the weight of the container weighed in advance, and dividing the resulting weight by the amount of the cultured broth.

The glutathione content was calculated by dividing the above-mentioned glutathione concentration by the cell concentration to give glutathione weight per dry cell (glutathione content).

As is clear from Table 1, the glutathione (reduced glutathione+oxidized glutathione) content was improved by enhancing the expression of ERV1 or ERO1 gene.

### [Comparative Example 1] Production of glutathione by yeast whose expression of GSH1 gene and GSH2 gene is enhanced

By a method similar to that in Example 1 except that recombinant yeast *Saccharomyces cerevisiae* YPH499/GSH1, GSH2 strain, whose expression of GSH1 and GSH2 is enhanced, which was obtained in Production Example 1, was used as a yeast, culturing was performed, and the weight of glutathione per dry cell was calculated. The results are shown in Table 1.

**Table 1**

| | strain | GSH content (wt %) | | |
|---|---|---|---|---|
| | | Reduced form | Oxidized form | Total (reduced + oxidized) |
| Comparative Example 1 | GSH1-enhanced + GSH2-enhanced strain | 1.91 | 0.73 | 2.64 |
| Example 1 | GSH1-enhanced + GSH2-enhanced + ERV1-enhanced strain | 1.96 | 0.85 | 2.81 |
| | GSH1-enhanced + GSH2-enhanced + ERO1-enhanced strain | 1.78 | 1.04 | 2.82 |

### [Example 2] Production of glutathione by strain in which expression of GSH1 gene and GSH2 gene is enhanced, expression of GLR1 gene is decreased, and expression of ERV1 gene or ERO1 gene is further enhanced (GSH1-enhanced + GSH2-enhanced + GLR1-destructed + ERV1-enhanced strain, GSH1-enhanced + GSH2-enhanced + GLR1-destructed + ERO1-enhanced strain)

The recombinant yeast YPH499/ΔGLR1, GSH1, GSH2, ERV1 strain and YPH499/ΔGLR1, GSH1, GSH2, ERO1 strain in which expression of GSH1 gene and GSH2 gene is enhanced, expression of GLR1 gene is decreased, and expression of ERV1 gene or ERO1 gene is further enhanced, which was obtained in Production Example 4, were subjected to seed culture by shaking in 5 ml of SD medium (6.7 g/L yeast nitrogen base w/o amino acids (manufactured by Difco laboratories) 20 g/L glucose) containing 0.5 µg/L aureobasidin A at 30°C for 16 - 24 hr.

Then, they were inoculated to erlenmeyer flask with baffles containing YPD medium (10 g/L yeast extract (manufactured by Difco laboratories), 20 g/L polypeptone (manufactured by Wako Pure Chemical Industries, Ltd.), 20 g/L glucose (manufactured by Nacalai Tesque)) (20 ml) at OD600=0.03, cultured under conditions of 30°C, agitation 150 rpm for 24 hr, and intracellular glutathione content was measured by the method described in Example 1.

### [Comparative Example 2] Production of glutathione by yeast in which expression of GSH1 gene and GSH2 gene is enhanced, and GLR1 gene is destructed

By a method similar to that in Example 2 except that *Saccharomyces cerevisiae* YPH499/ΔGLR1, GSH1, GSH2 strain, in which expression of GSH1 gene and GSH2 gene is enhanced, and GLR1 gene is destructed, which was obtained in Production Example 3, was used as a yeast, culturing was performed, and the weight of glutathione per dry cell was calculated. The results are shown in Table 2.

**Table 2**

| | strain | GSH content (wt %) | | |
|---|---|---|---|---|
| | | Reduced form | Oxidized form | Total (reduced + oxidized) |
| Example 1 | GSH1-enhanced + GSH2-enhanced + ERV1-enhanced strain | 1.96 | 0.85 | 2.81 |
| | GSH1-enhanced + GSH2-enhanced + ERO1-enhanced strain | 1.78 | 1.04 | 2.82 |
| Comparative Example 2 | GSH1-enhanced + GSH2-enhanced + GLR1-destructed strain | 1.43 | 1.23 | 2.66 |
| Example 2 | GSH1-enhanced + GSH2-enhanced + GLR1-destructed + ERV1-enhanced strain | 1.19 | 2.44 | 3.63 |
| | GSH1-enhanced + GSH2-enhanced + GLR1-destructed + ERO1-enhanced strain | 1.22 | 1.81 | 3.03 |

As is clear from Table 2, the glutathione content per cell was further improved by not only enhancing the expression of ERV1 gene or ERO1 gene but also destructing GLR1 gene.

### [Example 3] Production of glutathione by strain in which ERV1 is enhanced, expression of GSH1, GSH2 and YCF1 is further enhanced, and GLR1 gene is destructed (GGSH1-enhanced + GSH2-enhanced + ERV1-enhanced + YCF1-enhanced + GLR1-destructed strain)

The recombinant yeast YPH499/ΔGLR1, GSH1, GSH2, ERV1, YCF1 strain in which expression of ERV1 is enhanced, expression of GSH1, GSH2 and YCF1 is enhanced, and GLR1 gene is destructed, which was obtained in Production Example 5, was subjected to seed culture by shaking in 5 ml of SD medium (6.7 g/L yeast nitrogen base w/o amino acids (manufactured by Difco laboratories), 20 g/L glucose) containing 0.5 µg/L aureobasidin A at 30°C for 16 - 24 hr.

Then, they were inoculated to erlenmeyer flask with baffles containing YPD medium (10 g/L yeast extract (manufactured by Difco laboratories), 20 g/L polypeptone (manufactured by Wako Pure Chemical Industries, Ltd.), 20 g/L glucose (manufactured by Nacalai Tesque)) (20 ml) at OD600=0.03, cultured under conditions of 30°C, agitation 150 rpm for 24 hr, and intracellular glutathione concentration was measured by the method described in Example 1.

### [Comparative Example 3] Production of glutathione by yeast in which expression of GSH1, GSH2 and YCF1 is enhanced, and GLR1 gene is destructed

By a method similar to that in Example 3 except that *Saccharomyces cerevisiae* YPH499/ΔGLR1, GSH1, GSH2, YCF1 strain, in which expression of GSH1, GSH2 and YCF1 is enhanced, and GLR1 gene is destructed, which was obtained in Production Example 5, was used as a yeast, culturing was performed, and the weight of glutathione per dry cell was calculated. The results are shown in Table 3.

**Table 3**

| | strain | GSH content (wt %) | | |
|---|---|---|---|---|
| | | Reduced form | Oxidized form | Total (reduced + oxidized) (reduced + oxidized) |
| Example 2 | GSH1-enhanced + GSH2-enhanced + GLR1-destructed + ERV1-enhanced strain | 1.19 | 2.44 | 3.63 |
| Comparative Example 3 | GSH1-enhanced + GSH2-enhanced + GLR1-destructed + YCF1-enhanced strain | 1.90 | 1.65 | 3.55 |
| Example 3 | GSH1-enhanced + GSH2-enhanced + GLR1-destructed + ERV1-enhanced + YCF1-enhanced strain | 0.63 | 4.40 | 5.03 |

As is clear from Table 3, the glutathione content was further improved by enhancing the expression of both ERV1 gene and YCF1 gene.

### [Sequence Listing Free Text]

SEQ ID NO: 1: amino acid sequence of thiol oxidase (ERV1)
SEQ ID NO: 2: amino acid sequence of thiol oxidase (ERO1)
SEQ ID NO: 3: nucleotide sequence of nucleic acid encoding thiol oxidase (ERV1)
SEQ ID NO: 4: nucleotide sequence of nucleic acid encoding thiol oxidase (ERO1)
SEQ ID NO: 5: amino acid sequence of glutathione reductase (GLR1)
SEQ ID NO: 6: nucleotide sequence of nucleic acid encoding glutathione reductase (GLR1)
SEQ ID NO: 7: amino acid sequence of γ-glutamylcysteine synthase (GSH1)
SEQ ID NO: 8: nucleotide sequence of nucleic acid encoding γ-glutamylcysteine synthase (GSH1)
SEQ ID NO: 9: amino acid sequence of glutathione synthase (GSH2)
SEQ ID NO: 10: nucleotide sequence of nucleic acid encoding glutathione synthase (GSH2)
SEQ ID NO: 11: amino acid sequence of glutathione transport enzyme (YCF1)
SEQ ID NO: 12: nucleotide sequence of nucleic acid encoding glutathione transport enzyme (YCF1)
SEQ ID NO: 13: forward PCR primer for ERV1 gene amplification
SEQ ID NO: 14: reverse PCR primer for ERV1 gene amplification
SEQ ID NO: 15: forward PCR primer for ERO1 gene amplification
SEQ ID NO: 16: reverse PCR primer for ERO1 gene amplification
SEQ ID NO: 17: forward PCR primer for YCF1 gene amplification
SEQ ID NO: 18: reverse PCR primer for YCF1 gene amplification

This application is based on a patent application No. 2015-042909 filed in Japan (filing date: March 4, 2015), the contents of which are incorporated in full herein.

## Claims

1. A method of producing glutathione, comprising culturing a yeast whose thiol oxidase activity is increased as compared to the parent strain in a culture medium to produce glutathione, and recovering glutathione from the cultured broth obtained.

2. The production method according to claim 1, wherein the thiol oxidase is selected from the following (a) - (f):
(a) a protein consisting of the amino acid sequence shown by SEQ ID NO: 1 or 2,
(b) a protein having the amino acid sequence shown by SEQ ID NO: 1 or 2, wherein 1 or plural amino acids are deleted, substituted, inserted and/or added, and having a thiol oxidase activity,
(c) a protein consisting of an amino acid sequence having 60% or more sequence identity to the amino acid sequence shown by SEQ ID NO: 1 or 2,
(d) a protein consisting of an amino acid sequence encoded by a nucleotide sequence shown by SEQ ID NO: 3 or 4,
(e) a protein consisting of an amino acid sequence encoded by a DNA that hybridizes with a DNA having a nucleotide sequence complementary to SEQ ID NO: 3 or 4 under stringent conditions, and,
(f) a protein consisting of an amino acid sequence encoded by a DNA having the nucleotide sequence shown by SEQ ID NO: 3 or 4 wherein 1 or plural nucleotides are substituted, deleted, inserted and/or added, and having a thiol oxidase activity.

3. The production method according to claim 1 or 2, wherein the yeast is a yeast whose glutathione reductase activity is reduced as compared to the parent strain.

4. The production method according to claim 3, wherein the glutathione reductase is selected from the following (a) - (f):
(a) a protein consisting of the amino acid sequence shown by SEQ ID NO: 5,
(b) a protein having the amino acid sequence shown by SEQ ID NO: 5, wherein 1 or plural amino acids are deleted, substituted, inserted and/or added, and having a glutathione reductase activity,
(c) a protein consisting of an amino acid sequence having 60% or more sequence identity to the amino acid sequence shown by SEQ ID NO: 5,
(d) a protein consisting of an amino acid sequence encoded by a nucleotide sequence shown by SEQ ID NO: 6,
(e) a protein consisting of an amino acid sequence encoded by a DNA that hybridizes with a DNA having a nucleotide sequence complementary to SEQ ID NO: 6 under stringent conditions, and,
(f) a protein consisting of an amino acid sequence encoded by a DNA having the nucleotide sequence shown by SEQ ID NO: 6 wherein 1 or plural nucleotides are substituted, deleted, inserted and/or added, and having a glutathione reductase activity.

5. The production method according to any one of claims 1 to 4, wherein the yeast is a yeast in which the expression of γ-glutamylcysteine synthetase and/or glutathione synthetase is enhanced as compared to the parent strain.

6. The production method according to claim 5, wherein the γ-glutamylcysteine synthetase is selected from the following (a) - (f):
(a) a protein consisting of the amino acid sequence shown by SEQ ID NO: 7,
(b) a protein having the amino acid sequence shown by SEQ ID NO: 7, wherein 1 or plural amino acids are deleted, substituted, inserted and/or added, and having a γ-glutamylcysteine synthetase activity,
(c) a protein consisting of an amino acid sequence having 60% or more sequence identity to the amino acid sequence shown by SEQ ID NO: 7,
(d) a protein consisting of an amino acid sequence encoded by a nucleotide sequence shown by SEQ ID NO: 8,
(e) a protein consisting of an amino acid sequence encoded by a DNA that hybridizes with a DNA having a nucleotide sequence complementary to SEQ ID NO: 8 under stringent conditions, and,
(f) a protein consisting of an amino acid sequence encoded by a DNA having the nucleotide sequence shown by SEQ ID NO: 8 wherein 1 or plural nucleotides are substituted, deleted, inserted and/or added, and having a γ-glutamylcysteine synthetase activity.

7. The production method according to claim 5, wherein the glutathione synthetase is selected from the following (a) - (f) :
(a) a protein consisting of the amino acid sequence shown by SEQ ID NO: 9,
(b) a protein having the amino acid sequence shown by SEQ ID NO: 9, wherein 1 or plural amino acids are deleted, substituted, inserted and/or added, and having a glutathione synthetase activity,
(c) a protein consisting of an amino acid sequence having 60% or more sequence identity to the amino acid sequence shown by SEQ ID NO: 9,
(d) a protein consisting of an amino acid sequence encoded by a nucleotide sequence shown by SEQ ID NO: 10,
(e) a protein consisting of an amino acid sequence encoded by a DNA that hybridizes with a DNA having a nucleotide sequence complementary to SEQ ID NO: 10 under stringent conditions, and,
(f) a protein consisting of an amino acid sequence encoded by a DNA having the nucleotide sequence shown by SEQ ID NO: 10 wherein 1 or plural nucleotides are substituted, deleted, inserted and/or added, and having a glutathione synthetase activity.

8. The production method according to any of claims 1 to 7, wherein the yeast is a yeast in which the expression of glutathione transport enzyme is enhanced as compared to the parent strain.

9. The production method according to claim 8, wherein the glutathione transport enzyme is selected from the following (a) - (f):
(a) a protein consisting of the amino acid sequence shown by SEQ ID NO: 11,
(b) a protein having the amino acid sequence shown by SEQ ID NO: 11, wherein 1 or plural amino acids are deleted, substituted, inserted and/or added, and having a glutathione transport enzyme activity,
(c) a protein consisting of an amino acid sequence having 60% or more sequence identity to the amino acid sequence shown by SEQ ID NO: 11,
(d) a protein consisting of an amino acid sequence encoded by a nucleotide sequence shown by SEQ ID NO: 12,
(e) a protein consisting of an amino acid sequence encoded by a DNA that hybridizes with a DNA having a nucleotide sequence complementary to SEQ ID NO: 12 under stringent conditions, and,
(f) a protein consisting of an amino acid sequence encoded by a DNA having the nucleotide sequence shown by SEQ ID NO: 12 wherein 1 or plural nucleotides are substituted, deleted, inserted and/or added, and having a glutathione transport enzyme activity.

10. The production method according to any one of claims 1 to 9, wherein the yeast is a yeast belonging to the genus *Saccharomyces,* the genus *Candida* or the genus *Pichia.*

11. A method of producing reduced glutathione, comprising reducing oxidized glutathione in the glutathione obtained by the production method according to any one of claims 1 to 10.

12. A yeast having artificially modified genes such that the expression of thiol oxidase is enhanced and the expression of γ-glutamylcysteine synthetase and/or glutathione synthetase is enhanced as compared to the parent strain.

13. A yeast having artificially modified genes such that the expression of thiol oxidase is enhanced and the expression of glutathione transport enzyme is enhanced as compared to the parent strain.

14. The yeast according to claim 12 or 13, wherein the yeast is a yeast belonging to the genus *Saccharomyces,* the genus *Candida* or the genus *Pichia.*
